# EUROPEAN PATENT SPECIFICATION

(11) **EP 4 305 965 B1**
(45) Date of publication and mention of the grant of the patent: **06.08.2025**
(21) Application number: 23184820.1
(22) Date of filing: 11.07.2023
(51) Int. Cl.: A23C 9/00, A23C 9/14, A23J 1/20

(54) **DEVICES AND METHODS FOR PREPARING HUMAN MILK ORIGIN FORTIFIER COMPOSITIONS**
VORRICHTUNGEN UND VERFAHREN ZUR HERSTELLUNG VON VERSTÄRKUNGSZUSAMMENSETZUNGEN AUS MUTTERMILCH
DISPOSITIFS ET PROCÉDÉS DE PRÉPARATION DE COMPOSITIONS FORTIFIANTES D'ORIGINE LAITIÈRE HUMAINE

(30) Priority: 11.07.2022 CH 8292022
(43) Date of publication of application: 17.01.2024
(73) Proprietor: Babylat AG, 3008 Bern (CH)
(72) Inventor: YUDINA, Zinaida, 5610 Wohlen AG (CH); CRAMM, Georg, 4133 Pratteln (CH)
(74) Representative: Weickmann & Weickmann PartmbB

(56) References cited:
- EP-A1- 0 741 976
- EP-A2- 0 173 999
- WO-A1-2009/139624
- GB-A- 2 273 885

## Description

### Technical Field

The present invention relates to a device and a method for obtaining protein-enriched fractions from human milk.

### Background Art

Breastfeeding with breast milk is commonly regarded as very important for the development of newborns. Breast milk is uniquely adapted to the needs of human babies, providing them with the nutrients they need to grow and thrive, but at the same time incorporating a multitude of immunological, antibacterial and in general anti-pathogenic factors and epigenetic effects. Bioactive proteins can have enzymatic activity, enhance nutrient absorption, stimulate growth, modulate the immune system and assist in the defense against pathogens. Key bioactive proteins in human milk include lysozyme, α-lactalbumin, κ-casein and β-casein, as well as lactoferrin and immunoglobulins, especially secretory IgA (slgA).

Breast milk is the only known substance that has nourishing and at the same time immune-modulating functions for the child organism. The composition of breast milk changes over time, and there are suggestions that the nourishing function of breastfeeding in newborns decreases at the age of one year and above, and the immune-modulating function increases.

Still, breastfeeding rates are generally too low, especially in developed countries. Due to socio-economic conditions, and especially because of their professional activity, women often do not have enough time to fulfill the ideal breastfeeding criteria (continuous breastfeeding, i.e. breast milk with solid food) for up to two years and beyond. The World Health Assembly set a global target of increasing exclusive breastfeeding for infants under 6 months to at least 50% by 2025. Currently in developed countries the number is around 23%.

In order to close the gap between the actual breastfeeding rate and the physiological needs of babies, various infant formula milk products have been developed using cow's milk as a protein source and are currently commercially available. However, these products are inferior to breast milk in their physiological effects. In particular, they absolutely cannot fulfill the immunomodulatory functions of human breast milk.

A particular problem is revealed in premature infants, especially those having very low birth weight (VLBW, 1.5 kg or less as a birth weight). Also, they ideally need breast milk (from their mother or a donor). However, even the composition of human milk is not geared to the needs of premature babies. Premature babies need a higher protein content than babies born full-term. As a result of protein deficiency, there is a risk that premature babies are disturbed in their growth. To counteract this problem, cow's milk, donkey's milk or soy proteins are added to the donor milk in order to increase their nutritional value (so-called enrichment process). However, enrichment with proteins of non-human origin leads to risks, in particular for increased risk of gastrointestinal diseases, increased risks of infection, increased risk of allergies, and increased risk of necrotizing enterocolitis (NEC) - the leading mortality cause of preterm babies.

WO 2009/139624 discloses a process for producing milk fractions rich in secretory IgA by fractionating milk containing S-lgA.

EP 0173999 discloses a method for preparing milk products by precipitating caseins and subsequent filtration.

GB 2273885 discloses a method for lowering bacterial content of raw milk by dynamic microfiltration.

EP 0741976 is directed to a process for producing calcium supplemented cow milk including ultrafiltration or microfiltration.

Processes for obtaining protein-enriched fractions from breast milk are known and have been described, inter alia in EP 0 173 999 A2 (Biotest Pharma GmbH). The pH of the milk is reduced and the milk is filtered using a cross-flow filtration unit. Low-molecular weight components are removed from the resulting filtrate by a second cross-flow filtration. Isolation and/or enrichment of protein-rich fractions from milk is now commonly done by means of laboratory methods by appropriately trained professionals.

Unfortunately, the access to human milk origin fortifiers (HMOF) or equipment to produce human milk origin fortifiers is limited. Due to difficulties in sourcing and supply of human milk and the fortifiers produced therefrom, the production of human milk origin fortifiers remains primarily in the domain of commercial entities capable of supporting large-scale manufacturing sites. Furthermore, even when human milk fortifiers can be produced, sanitation and storage issues associated with human breast milk and human milk origin fortifiers, as well as lack of infrastructure to support distribution, preclude availability of human milk origin products having high, consistent quality over widespread geographic region.

Thus, there remains a need for improved systems and methods for preparing human-origin milk fortifiers that enable fortification of human breast milk, particularly an infant's own mother's milk, on-site where breast milk is pumped and/or at point of care, such as at a hospital or at home.

### Summary of the invention

The present invention relates to a device and a method as described in the claims. In some aspects, provided is a device for obtaining protein-enriched fractions from human or animal milk. In some aspects, the device is used on-site, simplifying the process of obtaining protein-enriched fractions from milk. In some aspects, provided is a device for protein enrichment of human breast milk. In some embodiments, the first filter is configured to provide a fat-enriched retentate and a fat-reduced filtrate from the breast milk.

Other advantageous embodiments and combinations of features come out from the detailed description below and the entirety of the claims.

### Brief description of the drawings

The drawings used to explain the embodiments show:
- Fig. 1A: depicts an exemplary schematic for two-step filtration for the preparation of human milk origin fortifier compositions;
- Fig. 1B: shows a SDS-PAGE gel using Coomassie staining for various stages of the two-step filtration illustrated in FIG. 1A;
- Fig. 2A, 2B: depict an exemplary device for the preparation of human milk origin fortifier compositions from human breast milk;
- Fig. 3A-3D, 4A-4C: depict the results of fat reduction or fat removal from human breast milk and subsequent protein enrichment using filters of various pore sizes and filter materials;

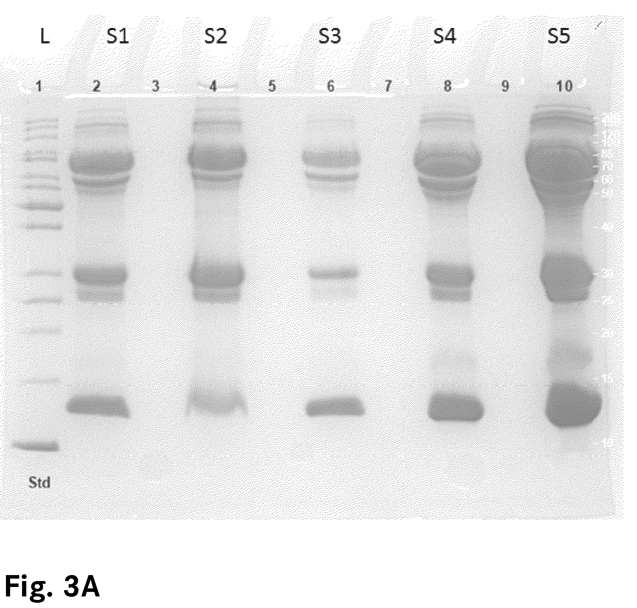

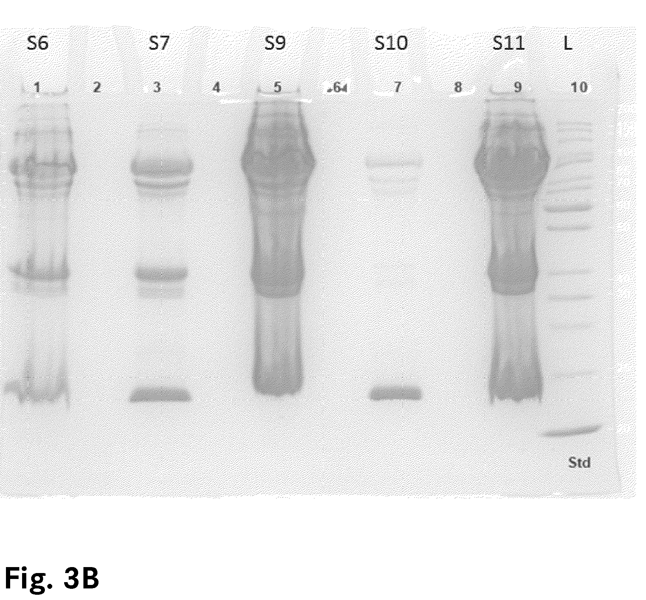

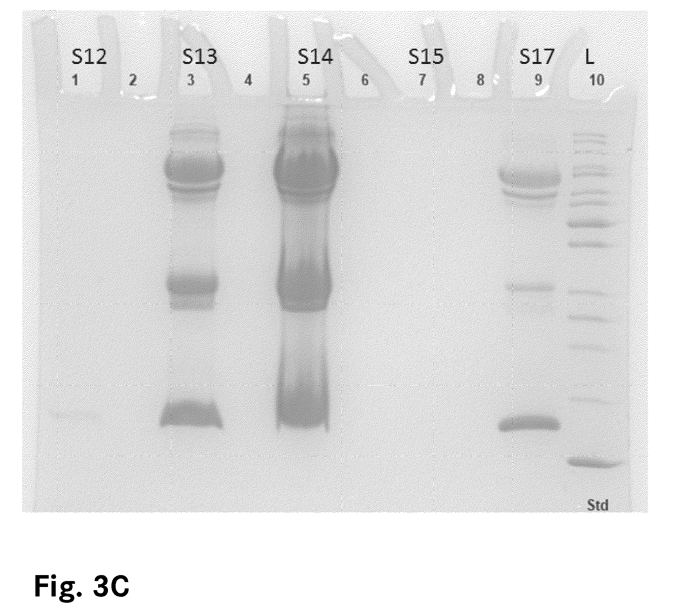

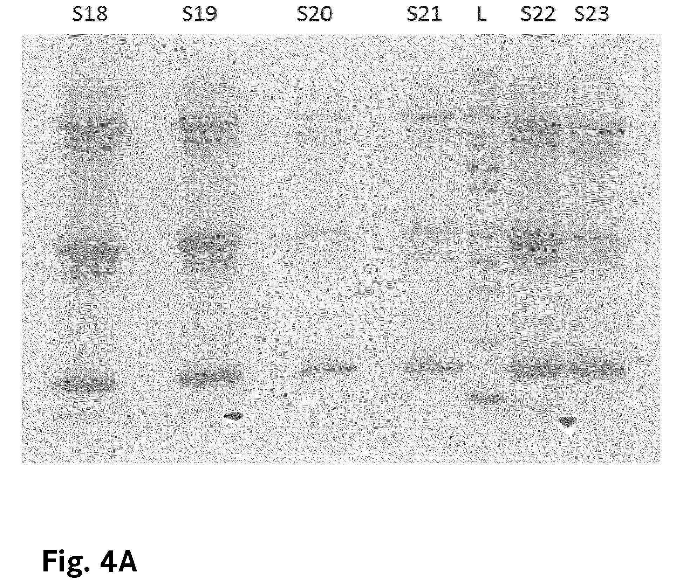

- Fig. 3A-3C, 4A-4B: show protein stained SDS-PAGE gels of initial milk samples, fat-enriched retentate, fat-reduced filtrate, and protein-enriched filtrate and protein reduced retentate samples obtained using various microfilters of differing pore size and filter materials; and

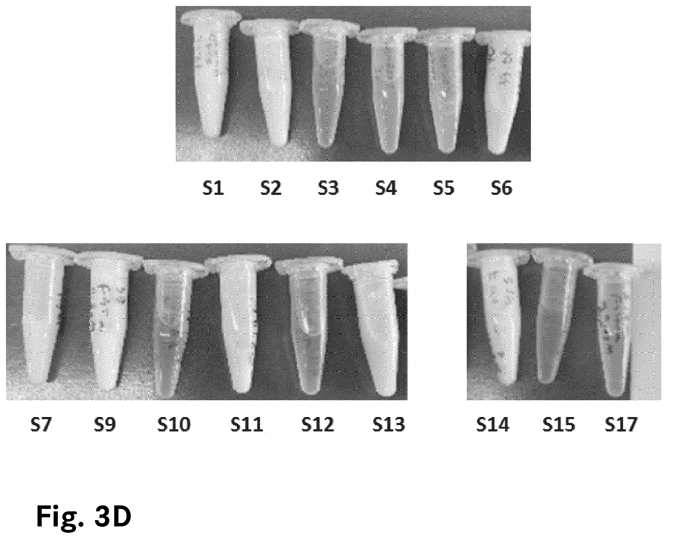

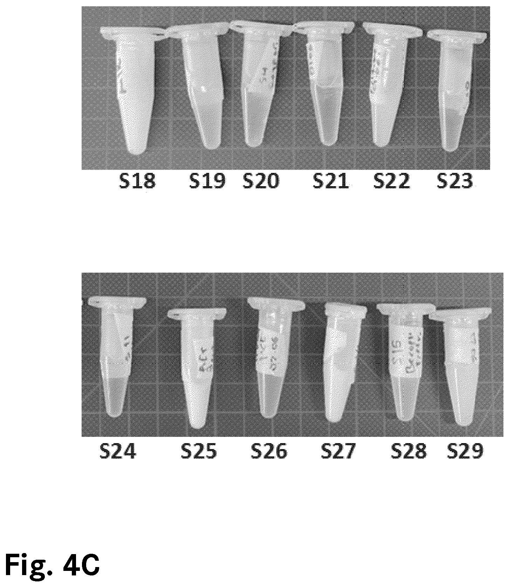

- Fig. 3D, 4C: show photographs of the initial milk, filtrated milk and various fat-enriched retentate, fat-reduced filtrate and protein-enriched retentate samples analyzed by SDS-PAGE in FIGS. 3A-3C and **FIG** 4A-4B.

In the figures, the same components are given the same reference symbols.

### Preferred embodiments

The following description sets forth exemplary methods, parameters and the like. It should be recognized, however, that such description is not intended as a limitation on the scope of the present disclosure but is instead provided as a description of exemplary embodiments.

The present invention provides an easy-to-use device or an easily performed method which allows the isolation of protein-enriched fractions of breast milk even by persons without laboratory technical knowledge, in particular by mothers and nurses. The present invention makes it possible, with a view to the optimal nutrition of preterm infants, to condition breast milk in its ingredients (in particular with regard to protein content and/or fat content). In some variations, the present invention gives mothers the opportunity to stock up on their fractions of breast milk to continue to provide for the infant even after breastfeeding has stopped. Also conceivable are other applications of the present invention, in particular in the field of recovery of breast milk or of fractions thereof by nurses.

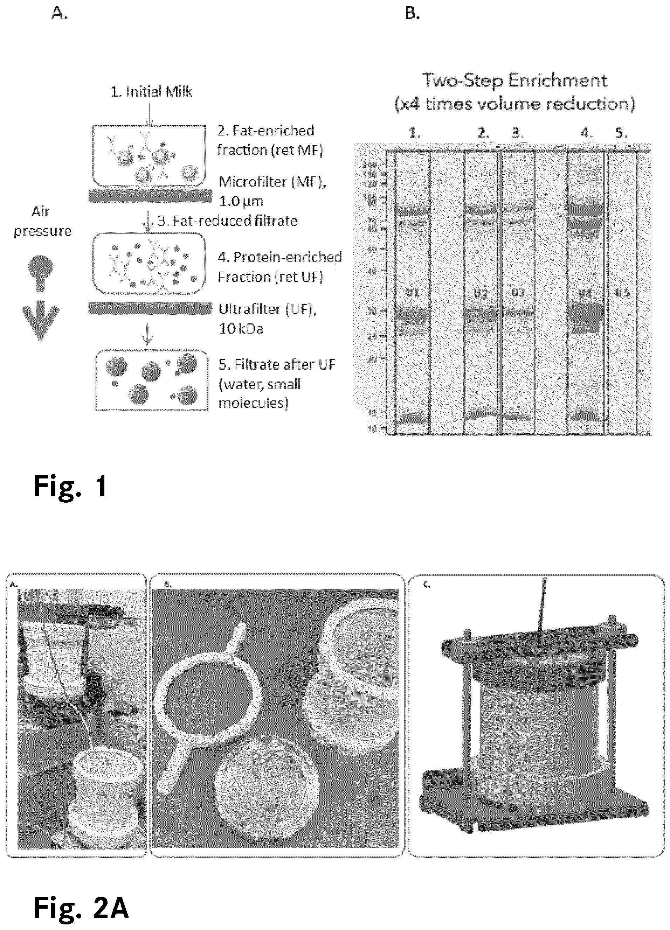

The devices and methods as described herein employ a two-step filtration system or process, as illustrated in FIG. 1A, to provide human milk origin fortifier compositions. With reference to FIG. 1A, an initial milk sample (labelled 1 in Fig 1A), such as breast milk pumped from an infant's own mother or a human donor, is provided. The initial human breast milk sample is passed through a first filtration system comprising a microfilter for the removal of fat. The microfilter separates fat from the proteins in the milk by preventing passage of lipids, to provide a fat-enriched retentate fraction (labelled 2 in Fig 1A) and a fat-reduced filtrate (labelled 3 in Fig 1A). The fat-reduced filtrate is further passed through a second filter in an ultrafiltration step to isolate proteins from water and small molecules (e.g., less than 10 kDa, such as sugars) present in the filtrate. The water and small molecules are able to pass through the ultrafiltration as a second filtrate (labelled 5 in Fig 1A), whereas the protein is concentrated in the protein-enriched retentate fraction (labelled 4 in Fig 1A). As shown in FIG. 1A, in one variation, positive air pressure may be applied to facilitate the filtration.

In contrast to existing methods used for the preparation of human milk origin fortifiers, the devices and methods of the present disclosure do not employ chemical means (e.g., acidification), thermal means (e.g., heating), or centrifugation to remove fat from the source human breast milk prior to concentration of the protein.

Instead, fat is removed from the initial breast milk by filtration, in particular by microfiltration. The resulting filtrate from microfiltration has substantially reduced lipid content; the resulting filtrate is subjected to subsequent filtration with an ultrafilter to concentrate the protein into a reduced volume of liquid. The reduction of the lipid content via microfiltration minimizes or avoids fouling of the ultrafilter for the increase of protein concentration and also minimizes or avoids high amounts of fat in the final fortifier.

In existing large scale processes, delipidation is usually based on centrifugation because it was assumed that filters would be immediately blocked by the lipids. Surprisingly, it has been found that the composition of human milk lipid micelles allows for an efficient filtering process. In one variation, the delipidation unit employs a cross-flow filtering process. In another variation, the delipidation unit employs a dead-end filtering process. This simplifies the process and the buildup of a corresponding device. The device is a functional unit that allows the recovery of protein-enriched fractions from the human or animal breast milk outside the laboratory. The device may be designed in such a way that its use does not require any professional laboratory knowledge, but may also be used by laypeople (especially mothers and nurses).

In some aspects, the device is configured to substantially reduce the final cost of human milk origin fortifiers (HMOF) compared with current industrial products. It makes HMOFs more available in developed countries and especially in developing ones, providing better outcome for preterm infants. Wide administration of HMOFs would reduce also the total duration of hospitalization of the infants, meaning less spending for hospitals. If hospitals prepare the protein fortifier from the milk, collected locally, it reduces the cost of the donor milk itself and thus can decrease the price of HMOF prepared using the device and method on site. Furthermore, the impact on the environment may be reduced because the product does not have to be shipped; it is prepared on site for local consumption.

In some variations, the protein-enriched retentate is suitable for administration to the premature or newborn after dilution with a suitable amount of human breast milk to match the infant's needed daily protein amount or for preservation in stock. The present invention may also be applied to the rearing of animals, including within the framework of zoological breeding programs.

The exemplary devices and methods provided herein help to substantially reduce the risk of losing proteins compared to the currently used long elaborative protocols with multiple steps.

### I. Device

In some aspects, provided is a device for protein enrichment of human breast milk. FIGS. 2A-2B illustrate an exemplary device for the two-step filtration of human breast milk to provide a human milk fortifier composition.

In one aspect, provided herein is a device for protein enrichment of human breast milk, comprising: a delipidation unit, comprising a first reservoir configured to receive breast milk, wherein the first reservoir is connected to a first filter; a first filter, wherein the first filter is in fluid connection with the first reservoir and is a cellulose filter using a pore size of 0.2 - 0.4 µm, wherein the first filter is configured to receive breast milk from the first reservoir and configured to provide a fat-enriched retentate and a fat-reduced filtrate from the breast milk; a pressure valve, wherein the pressure valve is in fluid connection with the first reservoir and the first filter, and wherein the pressure valve is configured to provide positive or negative air pressure to the delipidation unit; and an output wherein the output is configured to deliver the fat-reduced filtrate provided by the first filter; a protein-enrichment unit, wherein the protein-enrichment unit comprises: a second reservoir configured to receive the fat-reduced filtrate from the output of the delipidation unit; and a second filter, wherein the second filter has a nominal molecular weight limit of less than or equal to 100 kDa, and wherein the second filter is configured to provide a protein-enriched retentate and a protein-reduced filtrate.

In some embodiments, the device comprises two filtration units. In some embodiments, the device comprises a delipidation unit and a protein-enrichment unit. In some variations, the delipidation unit and the protein-enrichment unit are integrated as components of the device. In other variations, these units can also be configured as separate modules or modules that can be combined to form a structural unit. With reference to FIG. 2A, panel A, an exemplary device having two modular filtration units in fluid connection is depicted. In some embodiments, the device may be readily disassembled or assembled. With reference to FIG. 2A, panels B and C, an exemplary filtration unit is shown in various states of assembly.

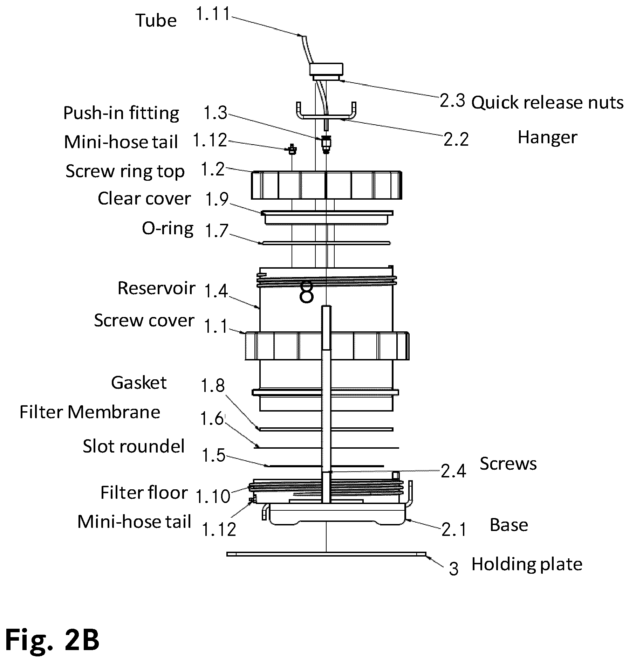

With reference to FIG. 2B, an exemplary filtration unit is shown in an exploded view. The single filtration unit as shown in FIG. 2B can be used as either a delipidation unit or a protein enrichment unit and might be transparent or have a transparent window depending upon the filter 1.6 and configuration of the inputs. At the top, a tube 1.11 is fed into a push-in fitting 1.3, which is inserted through a cover 1.9 and feeds into a reservoir 1.4. A second input line is shown at the mini-hose tail 1.12. Either of the tube 1.11 input or mini-hose 1.12 input can be configured as the input for source human breast milk (for a delipidation unit) or for fat-reduced filtrate (for a protein enrichment unit); the remaining tube 1.11 or mini-hose 1.12 input can be used as a pressure valve, for example, to provide positive air pressure to the filtration unit. The cover 1.9, which may be transparent or have a transparent window, is fitted against the reservoir with an O-ring 1.7. A screw ring top 1.2 is screwed onto the reservoir to hold the cover 1.9 and O-ring 1.7 in place. At the bottom of the filtration unit, a filter floor 1.10 is configured to hold a slot roundel 1.5 to increase hermeticity, a filter 1.6, and a gasket 1.8. The filter 1.6 is held in place at the bottom of the filtration unit and in fluid connection with the reservoir 1.4 by a second screw cover 1.1 which can be screwed securely into place with the filter floor 1.10. A second mini-hose tail 1.12 provides an output for fat-reduced filtrate (for a delipidation unit) or for protein-reduced filtrate (for a protein enrichment unit).

In some embodiments, the various components of the device, the delipidation unit and/or the protein enrichment unit may be assembled and held together by means suitable to provide air- and liquid-tight sealing. In some embodiments, the device, delipidation unit and/or the protein enrichment unit may comprise various screw fittings and/or clamp fittings to achieve fluid seal. In some embodiments, the output of the delipidation unit is connected to the second reservoir of the protein-enrichment unit through a tube connector.

With reference again to FIG. 2A, panel C, and FIG. 2B, the filtration unit may be further held together with a clamp or other compression mechanism suitable to prevent fluid leaks from the filtration unit. As shown in FIG. 2B, a clamp composed of a top hanger bar 2.2 together with quick-release nuts 2.3 form the moveable jaw of the clamp. The hanger 2.2 is held in place by a frame formed by at least two screws 2.4 which are mounted to a base 2.1, which forms the fixed jaw of the clamp. The screws 2.4 together with the quick-nuts 2.4 form can be tightened against the hanger bar 2.2 and the screw-ring top 1.2 to provide clamping.

As detailed above, the device as provided by the present disclosure is designed for use at point of care, whether in a hospital for use by care providers for a given patient or at home for use by a breast feeding or pumping parent. In contrast to large-scale commercial manufacturing and processing sites, the device provided herein is configured in size and operational requirements for benchtop use, for example, in a hospital clinical or laboratory setting, or a tabletop or countertop use in a house.

In some embodiments, the device may be characterized by its dimensions, or physical footprint. In some embodiments, the device has a physical footprint of less than or equal to about 4 cubic meters. In some embodiments, the device has a physical footprint of less than or equal to about 3 cubic meters. In some embodiments, the device has a physical footprint of less than or equal to about 2 cubic meters. In some embodiments, the device has a physical footprint of less than or equal to about 1.5 cubic meters. In some embodiments, the device has a physical footprint of less than or equal to about 1 cubic meter. In other embodiments, the device has a physical footprint of less than or equal to 0.5 cubic meter.

In other embodiments, the device may be characterized by the maximum volumetric capacity of source human breast milk that the device is capable of processing. In some embodiments, the device has a capacity of less than or equal to about 5 L, less than or equal to about 2.5 L, less than or equal to about 2 L, or less than or equal to about 1.5 L. In other embodiments, the device has a capacity of greater than or equal to about 100 mL, greater than or equal to about 200 mL, greater than or equal to about 300 mL, greater than or equal to about 500 mL, greater than or equal to about 1 L, greater than or equal to about 1.5 L. In some embodiments, the device has a capacity of less than or equal to about 2 L. In some embodiments, the device has a capacity of between about 100 mL and about 5 L, between about 100 mL and about 5 L, between about 100 mL and about 5 L, between about 100 mL and about 5 L, between about 100 mL and about 2.5 L, between about 500 mL and about 5 L, between about 500 mL and about 2 L or between about 500 mL and about 1.5 L. In some embodiments, the device has a capacity of between about 500 mL and about 1.5 L. In other embodiments, the device has a capacity of between about 500 mL and about 2 L.

As detailed herein, the device for protein enrichment of human breast milk on-site in a hospital or a residential setting. In view of the limited means for further purification, the device is self-contained and directly produces food safe human milk origin fortifier compositions without further purification. In some embodiments, the device is made of medical-grade or food-grade materials.

In some embodiments, the device is capable of processing at least about 2000 L of breast milk per device lifetime. In other embodiments, the device is capable of operating for at least about 1000 enrichment runs, optionally wherein the device processes between about 100 mL and about 2 L of source human breast milk per enrichment run.

In some embodiments, the device may be disassembled. In some embodiments, the device may be sterilized. In some embodiments, the device may be autoclaved. In some embodiments, the device is resistant to high temperatures, such as the temperatures experienced in an autoclave or dishwasher. In some embodiments, device is resistant to high temperatures, optionally wherein the high temperatures are less than or equal to about 70 degrees Celsius, optionally wherein the high temperatures are between about 50 and about 60 degrees Celsius. In some embodiments, device is resistant to high temperatures, optionally wherein the high temperatures are optionally wherein the high temperatures are less than or equal to about 135 degrees Celsius, optionally wherein the high temperatures are between about 120 and about 135 degrees Celsius.

In some aspects, provided is a device for protein enrichment of human breast milk. In some embodiments, the device for protein enrichment of human breast milk comprises at least two filtration units. In some embodiments, the device for protein enrichment of human breast milk comprises at least two filtration units, wherein one filtration unit of the at least two filtration units is a delipidation unit and the other filtration unit of the at least two filtration units is a protein-enrichment unit.

### a. Delipidation unit

In some embodiments, the delipidation unit comprises a first reservoir, a first filter, a pressure valve, and an output. In some embodiments, the first reservoir is configured to receive breast milk. In some embodiments, the first reservoir is in fluid connection with the first filter, and the first filter is configured to receive breast milk from the first reservoir. In some embodiments, the first filter is configured to provide a fat-enriched retentate and a fat-reduced filtrate from the breast milk.

In some embodiments, the delipidation unit may be disassembled. In some embodiments, the delipidation unit may be sterilized. In some embodiments, the delipidation unit may be autoclaved. In some embodiments, the delipidation unit is resistant to high temperatures, such as the temperatures experienced in an autoclave or dishwasher. In some embodiments, delipidation unit is resistant to high temperatures, optionally wherein the high temperatures are less than or equal to about 70 degrees Celsius, optionally wherein the high temperatures are between about 50 and about 60 degrees Celsius. In some embodiments, delipidation unit is resistant to high temperatures, optionally wherein the high temperatures are optionally wherein the high temperatures are less than or equal to about 135 degrees Celsius, optionally wherein the high temperatures are between about 120 and about 135 degrees Celsius.

In some embodiments, the delipidation unit comprises a first reservoir. In some embodiments, the delipidation unit comprises a first reservoir configured to receive breast milk. In some embodiments, the first reservoir is connected to a first filter as described herein. Such a reservoir in the delipidation unit may be employed to contain the full volume of source human breast milk to be converted to human milk origin fortifier composition and/or maintain hermetic sealing and sterility during fat removal from the human breast milk by the first filter. In contrast to various large-scale operational systems, the device as provided herein is designed for the concentration of human milk origin proteins at small volumes, such as those produced directly by a single lactating mother or other human milk donor.

In some embodiments, the first reservoir comprises glass and/or plastic. In some embodiments, the first reservoir comprises steel. In some embodiments, the first reservoir comprises steel and a transparent window comprising glass or plastic.

In some embodiments, the first reservoir may be characterized by its volumetric capacity. In some embodiments, the first reservoir has a capacity of less than or equal to about 5 L, less than or equal to about 2.5 L, less than or equal to about 2 L, or less than or equal to about 1.5 L. In other embodiments, the first reservoir has a capacity of greater than or equal to about 100 mL, greater than or equal to about 200 mL, greater than or equal to about 300 mL, greater than or equal to about 500 mL, greater than or equal to about 1 L, greater than or equal to about 1.5 L. In some embodiments, the first reservoir has a capacity of less than or equal to about 2 L. In some embodiments, the first reservoir has a capacity of between about 100 mL and about 5 L, between about 100 mL and about 5 L, between about 100 mL and about 5 L, between about 100 mL and about 5 L, between about 100 mL and about 2.5 L, between about 500 mL and about 5 L, between about 500 mL and about 2 L, between about 500 mL and about 1.5 L, between about 500 mL and about 1.5 L, between about 1 L and about 2 L, or between about 1.5 L and about 2 L.. In some embodiments, the first reservoir has a capacity of between about 500 mL and about 1.5 L. In other embodiments, the first reservoir has a capacity of between about 500 mL and about 2 L.

In some embodiments, the delipidation unit is configured to agitate breast milk in the first reservoir. In some embodiments, the delipidation unit is configured to stir breast milk in the first reservoir.

In some embodiments, the delipidation unit comprises a first filter. In some embodiments, the first filter is a microfilter. In some embodiments, the first filer is a microfilter. In some embodiments, the delipidation unit comprises a first filter wherein the first filter is configured to receive breast milk from the first reservoir and configured to provide a fat-enriched retentate and a fat-reduced filtrate from the breast milk.

The first filter may be characterized in terms of its pore size and/or its material composition. The filter pore size and material for the delipidation unit may vary, but the first filter is preferably suitable to allow proteins to pass through the first filter as part of the first filtrate, while minimizing the extent of protein loss due to lack of permeability through the first filter or protein absorption by the first filter. At the same time, the filter pore size and material for the delipidation unit should allow minimal fat or lipid fraction to pass through the first filter as part of the filtrate.

The first filter is characterized in terms of its pore size. The first filter has a pore size of 0.2 - 0.4 µm. In some embodiments, the first filter is a flat sheet filter. In some embodiments, the first filter is not a cartridge filter. In some embodiments, the first filter does not use tangential flow filtration. In some embodiments, the human breast milk is unprocessed before being passed through the first filter. In some embodiments, the human breast milk is not centrifuged. In some embodiments, the human breast milk is pasteurized before further processing.

In some variations, the first filter may be characterized by its material composition. As detailed above, the material for the first filter is selected to permit maximal passage of proteins and to minimize the passage of fats or lipids. In some variations of the foregoing, the material of the first filter is food grade, e.g., as determined by the Food and Drug Administration or other administrative bodies. In particular, in some variations, the material complies with regulation (EC) No. 1935/2004 (Art. 3).

In some embodiments, the first filter comprises one layer. In some embodiments, the first filter comprises a first layer and a second layer. In certain variations, the first layer comprises glass fiber, and the second layer comprises nitrocellulose. In some embodiments, the second layer is configured to remove glass fibers.

In some embodiments, the first filter is removable from the delipidation unit. In some embodiments, the first filter is replaceable from the delipidation unit. In some embodiments, the first filter is a single use filter.

In some embodiments, the delipidation unit is configured to hold a first filter having a specific physical dimensions, such as diameter and/or thickness. In some embodiments, the delipidation unit is configured to hold a first filter having a diameter between about 140 mm and about 160 mm, between about 140 mm and about 150 mm, between about 145 mm and about 150 mm, between about 147 mm and about 150 mm, or between about 147 mm and about 148 mm. In some embodiments, the delipidation unit is configured to hold a first filter having a diameter between about 70 mm and about 140 mm, between about 75 mm and about 140 mm, or between about 76 mm and about 140 mm. In other embodiments, the delipidation unit is configured to hold a first filter having a thickness of between about 1 mm and about 1mm, between about 1 mm and about 5 mm, between about 2 mm and about 10 mm, between about 2 mm and about 7 mm, between about 2 mm and about 5 mm, between about 5 mm and about 10 mm, between about 5 mm and about 7 mm, or between about 7 mm and about 10 mm.

In some variations, it may be desirable for the air pressure of the delipidation unit to be adjusted, either for positive or negative pressure, to expedite filtration for fat removal from the source human breast milk. In some variations, it may be desirable for the positive air pressure of the delipidation unit to be adjusted, to expedite filtration for fat removal from the source human breast milk. In some embodiments, the delipidation unit comprises a pressure valve. In some embodiments, the pressure valve is in fluid connection with the first reservoir and the first filter. In some embodiments, the pressure valve is configured to provide positive or negative air pressure to the delipidation unit. In some embodiments, the pressure valve is configured to provide positive or negative air pressure to the delipidation unit. In some embodiments, the pressure valve is configured to provide positive air pressure generated by a peristaltic pump.

In some embodiments, the pressure valve is configured to supply between about 0.5 and about 4 bar of absolute pressure to the first reservoir and first filter. In some embodiments, the pressure valve is configured to supply between about 1 and about 4 bar of absolute pressure to the first reservoir and first filter. In some embodiments, the pressure valve is configured to supply between about 2 and about 4 bar of absolute pressure to the first reservoir and first filter. In some embodiments, the pressure valve is configured to supply between about 3 and about 4 bar of absolute pressure to the first reservoir and first filter. In some embodiments, the pressure valve is configured to supply less than or equal to about 4 bar of absolute pressure to the first reservoir and first filter. In some embodiments, the pressure valve is configured to provide pressure from a peristaltic pump. It should be recognized that the device as described herein may include additional components suitable to enable pressurization or de-pressurization of the delipidation unit, including but not limited to sealing (O) rings, gaskets, clamps, quick release clamps, valves, tubing/hose(s), Swagelok connections, ferrules, etc.

In addition, in some embodiments, the pressure valve may further comprise an air filter, for example, to avoid contamination of the source human breast milk, the fat-enriched retentate or the fat-reduced filtrate.

In some embodiments, the delipidation unit may be characterized by its maximum pressure tolerance. In some embodiments, the delipidation unit is configured to withstand between about 1 and about 4 bar of absolute pressure. In some embodiments, the delipidation unit is configured to withstand between about 2 and about 4 bar of absolute pressure. In some embodiments, the delipidation unit is configured to withstand between about 3 and about 4 bar of absolute pressure. In some embodiments, the delipidation unit is configured to withstand less than or equal to about 6 bar of absolute pressure. In some embodiments, the delipidation unit is configured to withstand less than or equal to about 4 bar of absolute pressure.

In some embodiments, the delipidation unit comprises an output. In some embodiments, the output is configured to deliver the fat-reduced filtrate provided by the first filter. In some embodiments, the output of the delipidation unit is capable of being configured or is configured for unidirectional flow, such that any liquid passed through the device proceeds from the first reservoir, through the first filter, and exits from the output into the protein-enrichment unit with minimal or no reverse flow of the fat-reduced filtrate back into the first reservoir.

### b. Protein enrichment unit

In some embodiments, the device comprises a protein-enrichment unit. In some embodiments, the protein-enrichment unit comprises a second reservoir and a second filter. In some embodiments, the second reservoir is configured to receive the fat-reduced filtrate from the output of the delipidation unit.

In some embodiments, the protein enrichment unit may be disassembled. In some embodiments, the protein enrichment unit may be sterilized. In some embodiments, the protein enrichment unit may be autoclaved. In some embodiments, the protein enrichment unit is resistant to high temperatures, such as the temperatures experienced in an autoclave or dishwasher. In some embodiments, protein enrichment unit is resistant to high temperatures, optionally wherein the high temperatures are less than or equal to about 70 degrees Celsius, optionally wherein the high temperatures are between about 50 and about 60 degrees Celsius. In some embodiments, protein enrichment unit is resistant to high temperatures, optionally wherein the high temperatures are optionally wherein the high temperatures are less than or equal to about 135 degrees Celsius, optionally wherein the high temperatures are between about 120 and about 135 degrees Celsius.

In some embodiments, the protein enrichment unit comprises a second reservoir. In some embodiments, the protein enrichment unit comprises a second reservoir, configured to receive the fat-reduced filtrate from the output of the delipidation unit. A second reservoir may be provided in fluid connection with an output of the delipidation unit. The second reservoir is configured to receive the fat-reduced filtrate generated by the delipidation unit. It should be recognized that, due to the removal of fat, volume of the fat-reduced filtrate is expected be less than or equal to that of the initial volume of source human breast milk delivered into the first reservoir of the delipidation unit.

In some embodiments, the second reservoir may be characterized by the material out of which it is made or its physical properties. In some embodiments, the second reservoir comprises glass and/or plastic. In some embodiments, the second reservoir comprises steel. In some embodiments, the second reservoir comprises steel and a transparent window comprising glass or plastic.

In some embodiments, the second reservoir may be characterized by its volumetric capacity. In some embodiments, the second reservoir has a capacity of less than or equal to about 5 L, less than or equal to about 2.5 L, less than or equal to about 2 L, or less than or equal to about 1.5 L. In other embodiments, the second reservoir has a capacity of greater than or equal to about 100 mL, greater than or equal to about 200 mL, greater than or equal to about 300 mL, greater than or equal to about 500 mL, greater than or equal to about 1 L, greater than or equal to about 1.5 L. In some embodiments, the second reservoir has a capacity of less than or equal to about 2 L. In some embodiments, the second reservoir has a capacity of between about 100 mL and about 5 L, between about 100 mL and about 5 L, between about 100 mL and about 5 L, between about 100 mL and about 5 L, between about 100 mL and about 2.5 L, between about 500 mL and about 5 L, between about 500 mL and about 2 L, between about 500 mL and about 1.5 L, between about 1 L and about 2 L, or between about 1.5 L and about 2 L. In some embodiments, the second reservoir has a capacity of between about 500 mL and about 1.5 L. In other embodiments, the second reservoir has a capacity of between about 500 mL and about 2 L.

In some embodiments, the protein-enrichment unit is configured to agitate the fat-reduced filtrate in the second reservoir. In some embodiments, the protein-enrichment unit is configured to stir fat-reduced filtrate in the second reservoir.

In some embodiments, the protein-enrichment unit comprises a second filter. In some embodiments, the second filter is an ultrafilter. In some embodiments, the second filter is an ultrafiltration filter. In some embodiments, the protein-enrichment unit comprises a second filter, wherein the second filter is configured to provide a protein-enriched retentate and a protein-reduced filtrate.

The first filter may be characterized in terms of its nominal molecular weight limit (NMWL) and/or its material composition. The filter NMWL and material for the protein-enrichment unit may vary, but the second filter is preferably suitable to allow water and small molecules such as sugars to pass through the second filter as part of the protein-reduced filtrate, while preventing the passage of protein into and through the second filter. Due to the selective permeation of water and small molecules through the second filter as compared to the protein, protein originally present in the fat-reduced filtrate becomes concentrated in a protein-enriched retentate.

In some embodiments, the second filter may be characterized in terms of its nominal molecular weight limit (NMWL). As described herein, a filter having a nominal molecular weight limit permits passage of molecules having a molecular weight less than or equal to the NMWL value and block passage of molecules having a molecular weight greater than the NMWL value. In some embodiments, the second filter has a nominal molecular weight limit of less than or equal to 100 kDa, less than or equal to about 80 kDa, less than or equal to about 75 kDa, less than or equal to about 60 kDa, less than or equal to about 50 kDa, less than or equal to about 40 kDa, less than or equal to about 25 kDa, or less than or equal to about 20 kDa. In other embodiments, the second filter has a nominal molecular weight limit of at least about 2 kDa, at least about 5 kDa, at least about 10 kDa, at least about 15 kDa, at least about 20 kDa, at least about 25 kDa, or at least about 50 kDa. In some embodiments, the second filter is an ultrafilter having a nominal molecular weight limit between about 2 kDa and about 100 kDa, between about 2 kDa and about 50 kDa, between about 2 kDa and about 10 kDa, between about 5 kDa and about 100 kDa, between about 5 kDa and about 10 kDa, between about 4 kDa and about 10 kDa, or between about 10 kDa and about 100 kDa. In some embodiments, the second filter is an ultrafilter less than or equal to about 100 kDa. In some embodiments, the second filter is an ultrafilter having a nominal molecular weight limit of at least about 10 kDa.

In some variations, the second filter may be characterized by its material composition. As detailed above, the material for the second filter is selected to concentrate proteins in the retentate by minimizing permeation and loss of proteins while allowing passage of water and small molecules with molecular weights less than the NMWL. In some embodiments, the second filter is composed of food grade or medical grade material. In some variations, suitable materials for the second filter may include but are not limited to cellulose or derivatives thereof (e.g., nitrocellulose), or polymers (e.g., polycarbonate, polyvinylidene fluoride, or polyethersulfone). In certain variations, the second filter comprises polyethersulfone. In some variations of the foregoing, the material of the first filter is food grade, e.g., as determined by the Food and Drug Administration or other administrative bodies. In particular, in some variations, the material complies with regulation (EC) No. 1935/2004 (Art. 3).

In some embodiments, the second filter is removable from the protein enrichment unit. In some embodiments, the second filter is replaceable from the protein enrichment unit. In some embodiments, the second filter is a single use filter.

In some embodiments, the protein enrichment unit is configured to hold a second filter having a specific physical dimensions, such as diameter and/or thickness. In some embodiments, the protein enrichment unit is configured to hold a second filter having a diameter between about 140 mm and about 160 mm, between about 140 mm and about 150 mm, between about 145 mm and about 150 mm, between about 147 mm and about 150 mm, or between about 147 mm and about 148 mm. In other embodiments, the protein enrichment unit is configured to hold a second filter having a thickness of between about 1 mm and about 1mm, between about 1 mm and about 5 mm, between about 2 mm and about 10 mm, between about 2 mm and about 7 mm, between about 2 mm and about 5 mm, between about 5 mm and about 10 mm, between about 5 mm and about 7 mm, or between about 7 mm and about 10 mm.

It should be recognized that the device as described herein may include additional components suitable to enable insertion and/or removal of the first filter unit as well as assembly and/or disassembly of the delipidation unit, including but not limited to sealing (O) rings, gaskets, clamps, quick release clamps, valves, tubing/hose(s), Swagelok connections, ferrules, etc. Similarly, it should be recognized that the device as described herein may include additional components suitable to enable insertion and/or removal of the second filter as well as assembly and/or disassembly of the protein enrichment unit, including but not limited to sealing (O) rings, gaskets, clamps, quick release clamps, valves, tubing/hose(s), Swagelok connections, ferrules, etc.

In some embodiments, the device requires source human breast milk as the sole input. In some embodiments, the device requires source human breast milk and positive air pressure as the sole inputs. In some embodiments, the device does not require the addition of aggressive solutions such as NaOH or HCl. In some embodiments, the device may process 0.5-2 L of human breast milk per use. In some embodiments, the size of the device allows for easy tabletop assembly and handwashing by the final user, for example by nurses.

In some variations, it may be desirable to apply positive or negative pressure to the protein enrichment unit in order to initiate and/or accelerate filtration. In one variation, it may be desirable to apply positive pressure to the protein enrichment unit in order to initiate and/or accelerate filtration. In some variations, the protein enrichment unit comprises a second pressure valve configured to be opened, closed and/or operated independently of the pressure valve in the delipidation unit. In some embodiments, the device comprises a second pressure valve. In some embodiments, the second pressure valve is in fluid connection with the second reservoir and the second filter. In some embodiments, the second pressure valve is configured to provide positive air pressure to the protein enrichment unit. In some embodiments, the second pressure valve is configured to provide negative air pressure to the protein enrichment unit. In some embodiments, the second pressure valve is configured to provide positive or negative air pressure to the protein enrichment unit. In some embodiments, the second pressure valve is configured to provide pressure from a peristaltic pump.

In some embodiments, the second pressure valve is configured to supply between about 0.5 and about 4 bar of absolute pressure to the second reservoir and second filter. In some embodiments, the second pressure valve is configured to supply between about 1 and about 4 bar of absolute pressure to the second reservoir and second filter. In some embodiments, the second pressure valve is configured to supply between about 2 and about 4 bar of absolute pressure to the second reservoir and second filter. In some embodiments, the second pressure valve is configured to supply between about 3 and about 4 bar of absolute pressure to the first reservoir and first filter. In some embodiments, the second pressure valve is configured to supply less than or equal to about 4 bar of absolute pressure to the second reservoir and second filter. In some embodiments, the second pressure valve is configured to provide pressure from a peristaltic pump. It should be recognized that the device as described herein may include additional components suitable to enable pressurization or de-pressurization of the delipidation unit, including but not limited to sealing (O) rings, gaskets, clamps, quick release clamps, valves, tubing/hose(s), Swagelok connections, ferrules, etc.

In addition, in some embodiments, the second pressure valve may further comprise an air filter, for example, to avoid contamination the source the fat-reduced filtrate, the protein-enriched retentate, the protein-reduced filtrate.

It should be recognized that the device as described herein may include additional components suitable to enable pressurization or de-pressurization of the protein-enrichment unit, including but not limited to sealing (O) rings, gaskets, clamps, quick release clamps, valves, tubing/hose(s), Swagelok connections, ferrules, etc.

In some embodiments, the protein-enrichment unit may be characterized by its maximum pressure tolerance. In some embodiments, the protein-enrichment unit is configured to withstand between about 0.5 and about 4 bar of absolute pressure. In some embodiments, the protein-enrichment unit is configured to withstand between about 2 and about 4 bar of absolute pressure. In some embodiments, the protein-enrichment unit is configured to withstand between about 3 and about 4 bar of absolute pressure. In some embodiments, the protein-enrichment unit is configured to withstand less than or equal to about 6 bar of absolute pressure. In some embodiments, the protein-enrichment unit is configured to withstand less than or equal to about 4 bar of absolute pressure.

In some embodiments, the protein enrichment unit further comprises a second output. In some embodiments, the protein enrichment unit further comprises a second output, wherein the second output is configured to deliver the protein-reduced filtrate. In some embodiments, the protein enrichment unit further comprises a second output and a third reservoir. In some embodiments, the second output is configured to deliver the protein-reduced filtrate to the third reservoir.

In some embodiments, the third reservoir comprises glass and/or plastic. In some embodiments, the third reservoir comprises steel. In some embodiments, the third reservoir comprises steel and a transparent window comprising glass or plastic.

In some embodiments, the third reservoir may be characterized by its volumetric capacity. In some embodiments, the third reservoir has a capacity of less than or equal to about 5 L, less than or equal to about 2.5 L, less than or equal to about 2 L, or less than or equal to about 1.5 L. In other embodiments, the third reservoir has a capacity of greater than or equal to about 100 mL, greater than or equal to about 200 mL, greater than or equal to about 300 mL, greater than or equal to about 500 mL, greater than or equal to about 1 L, greater than or equal to about 1.5 L. In some embodiments, the third reservoir has a capacity of less than or equal to about 2 L. In some embodiments, the third reservoir has a capacity of between about 100 mL and about 5 L, between about 100 mL and about 5 L, between about 100 mL and about 5 L, between about 100 mL and about 5 L, between about 100 mL and about 2.5 L, between about 500 mL and about 5 L, between about 500 mL and about 2 L, between about 500 mL and about 1.5 L, between about 500 mL and about 1.5 L, between about 1 L and about 2 L, or between about 1.5 L and about 2 L.. In some embodiments, the third reservoir has a capacity of between about 500 mL and about 1.5 L. In other embodiments, the third reservoir has a capacity of between about 500 mL and about 2 L.

### c. Device Configuration and Operation

In some embodiments, the device has an operation time of about 10 hours per filtration. In some embodiments, the device has an operation time of about 1 hour to about 10 hours per filtration. In some embodiments, the device has an operation time of about 1 hour to about 20 hours per filtration.

Both the delipidation unit as well as the protein enrichment unit may be run under controlled temperature, e.g. in a controlled temperature environment. In some variations wherein the device is operated under a controlled temperature, the protein recovery and/or integrity may be higher due to reduced levels of denaturation.

In some embodiments, the device is configured to maintain a temperature of at least about 0 degrees Celsius, at least about 2 degrees Celsius, at least about 4 degrees Celsius, at least about 5 degrees Celsius, at least about 7 degrees Celsius or at least about 10 degrees Celsius. In other embodiments, the device is configured to maintain a temperature of less than or equal to about 16 degrees Celsius, less than or equal to about 15 degrees Celsius, less than or equal to about 14 degrees Celsius, less than or equal to about 12 degrees Celsius, or less than or equal to about 10 degrees Celsius. In some embodiments, the device is configured to maintain a temperature of between about 0 and about 16 degrees Celsius, between about 0 and about 10 degrees Celsius, between about 4 and about 10 degrees Celsius, between about 4 and about 16 degrees Celcius, between about 5 and about 16 degrees Celsius, between about 5 and about 10 degrees Celsius, between about 10 and about 16 degrees Celsius, or between about 10 and about 15 degrees Celsius. In some embodiments, the device is configured to maintain a temperature of between 0 and 10 degrees Celsius. In some embodiments, the device is configured to maintain a temperature of between 0 and 16 degrees Celsius. In some embodiments, the device is configured to maintain a temperature of between 5 and 10 degrees Celsius. In some embodiments, the device is configured to maintain a temperature of about 16 degrees Celsius. In some embodiments, the device is configured to maintain a temperature of less than about 16 degrees Celsius.

In some embodiments, the delipidation unit and protein enrichment unit may be configured to maintain a controlled temperature independently of one another.

In some embodiments, the delipidation unit and protein enrichment unit are configured to be or capable of being sealed without the use of an external agent, such as a sealant, lubricant, or tape. In some embodiments, the delipidation unit and protein enrichment unit are configured to be hermetically sealed. In some embodiments, the delipidation unit and protein enrichment unit are hermetically sealed. In some embodiments, the first reservoir is glass. In some embodiments, the first reservoir is plastic. In some embodiments, the second reservoir is glass. In some embodiments, the second reservoir is plastic. It should be recognized that the device as described herein may include additional components suitable to enable operation, including but not limited to sealing (O) rings, gaskets, clamps, quick release clamps, valves, tubing/hose(s), Swagelok connections, ferrules, etc.

In some embodiments, the device may be characterized by its maximum pressure tolerance. In some embodiments, the device is configured to withstand between about 1 and about 4 bar of absolute pressure. In some embodiments, the device is configured to withstand between about 2 and about 4 bar of absolute pressure. In some embodiments, the device is configured to withstand between about 3 and about 4 bar of absolute pressure. In some embodiments, the device is configured to withstand less than or equal to about 6 bar of absolute pressure. In some embodiments, the device is configured to withstand less than or equal to about 4 bar of absolute pressure.

In some embodiments, the present disclosure provides a device as described herein and instructions for use, including but not limited to assembly, disassembly, operation, cleaning and maintenance, etc.

### II. Methods of Preparing Human Milk Fortifier

The method comprises passing human breast milk through a first filter and producing a fat-reduced filtrate, then passing the fat-reduced filtrate through a second filter and collecting a protein-enriched retentate, wherein the method does not employ chemical means, thermal means or centrifugation to remove fat from human breast milk prior to concentration of the protein.

In some embodiments, the temperature of the human breast milk is not raised above 25 °C. In some embodiments, the temperature of the human breast milk is not raised above 16 °C. In some embodiments, the temperature of the human breast milk is between 5 and 16 °C during at least one filtering step. In some embodiments, the temperature of the human breast milk is between 5 and 16 °C during both filtering steps.

In some embodiments, the method comprises agitating or stirring the human breast milk, the fat-reduced filtrate and/or the fat-enriched retentate.

In some embodiments, the method further comprises estimating the dilution ratio of the protein-enriched retentate to the source human breast milk. In some embodiments, the estimate is for use in feeding a neonate. In some embodiments, the estimate is for use in determining a final feeding volume and/or individual growth rate of the neonate.

In some embodiments, a total volume of the human milk fortifier obtained from an initial volume of the human or animal milk is less than a fifth of the initial volume. In some embodiments, the volume of the human milk fortifier is between 10 and 20 times less than the volume of the human breast milk. In some embodiments, the protein concentration of the human milk fortifier is between 5 and 20 times more than the protein concentration of the human breast milk.

### III. Human Milk Fortifier Compositions and Methods of Use thereof

In some embodiments, the device as provided herein may also provide improved recovery of protein content in the final human milk fortifier.

The human milk fortifier compositions as provided herein may be liquid. Human milk fortifier compositions may be characterized by a liquid volume or a relative reduction in volume, such as by a concentration factor, as compared to the volume of source human breast milk from which it was obtained.

The human milk fortifier compositions may be described by one or more components present in the human milk fortifier composition, including but not limited to fat, protein, sugar, and/or water. The human milk fortifier compositions may be characterized by the content of one or more components by relative content of a given component per unit weight (for example, per gram or kilogram) or per unit volume (for example, per milliliter or liter) of the total human milk fortifier content. For infants, the dosage is sensibly indicated in gram of protein/kg/day. The human milk fortifier compositions may be characterized by the content of one or more components by relative content of a given component as compared to the content of the same component(s) present in the source human breast milk from which the fortifier composition was obtained or derived.

Human milk fortifier composition may be characterized by its protein content. In contrast to large-scale operations using pooled human breast milk in industrial scale systems, which result in significant losses of protein, sometimes up to 90% or higher, the devices and methods of the present disclosure allow for higher recovery of protein content-that is, higher protein yield-from the source human breast milk.

In some embodiments, the device and method of the present disclosure allow for recovery of at least about 25%, at least about 30%, at least about 35%, at least about 40%, at least about 45%, or at least about 50% protein content from the source human breast milk. In some embodiments, the composition retains over 50% of the protein mass of the source human breast milk.

The human milk fortifier composition may be characterized by the distribution or profile of protein classes or individual proteins present in the overall protein content. The human milk fortifier composition may comprise the same protein content profile as the source human breast milk from which it was obtained.

Human milk fortifier composition may be characterized by its immunological activity or the presence of immunological proteinsThe composition may retain the immunoactivity of the source human breast milk. The immunoactivity may provide protection against one or more medical conditions. The immunoactivity may protect against allergy, late-onset sepsis, necrotizing enterocolitis, or a combination thereof.

A composition according to the above description may be feeded to a neonate. Ideally, the neonate does not experience unwanted or dangerous side effects associated with heterologous fortifiers obtained from animal milk.

The neonate may be a full-term neonate. The full-term neonate may have been born after at least 37 weeks of gestation. The neonate may be a preterm neonate. A preterm neonate may have been born less than 37 weeks of gestation.

The neonate may be a neonate having very low birth weight (VLBW). The neonate may have a birth weight of less than or equal to about 1.5 kilograms. The neonate may have a weight of less than or equal to about 2.5 kilograms. In some embodiments, the neonate has a weight of less than or equal to about 3.5 kilograms.

The diet of the neonate does not comprise non-human milk products. The neonate may be regularly fed the human milk fortifier for at least about one month, at least about two months, at least about three months, at least about four months, or at least about six months.

The human milk fortifier composition is produced from the breast milk of the neonate's own mother. In some embodiments, the human breast milk of the composition is not produced by the mother of the neonate. Human milk fortifier composition may be produced from the breast milk of a donor, who is not the neonate's own mother.

The neonate may be fed between about five and about ten small doses of human milk fortifier per day, totaling to about 10 ml per day of protein fortifier. In some embodiments, 100ml of the protein fortifier at a final protein concentration of 100mg/ml are obtained from a single enrichment procedure, from an initial volume of human milk of 2.0l.

After enrichment, it is recommended to aliquote the total volume (of about 100ml) of enriched protein fraction to the small aliquotes, 10 ml each. The 10ml aliquote is an average daily portion of the fortifier which we recommend to dilute by human milk in different ratios based on the daily protein needs of the neonate (calculated by g of protein/kg and being about 4g of protein/kg(day)). The daily portion of the fortifier consumed by the preterm neonate can vary from 3.0 to 10.0ml of the fortifier and depends from the weight gain of the neonate, his or her gestational age and total volume of the milk he or she could consume daily.

Thus the dosage of the fortifier and the ratio of its dilution by human milk will be adapted to the individual requirements of the neonate. In an early stage, when neonates can not consume large volumes of the milk, the role of fortification is very high (milk that is more concentrated by protein is required to cover the growth of the neonate). In this case, small dilution factors of fortifier by milk (e. g. 1:4 in the final volumes of the fortifier to the human milk) will be chosen. In later stages, when the neonate starts to consume a larger volume of milk, the role of the fortification is decreased since the needed daily amount of proteins required for optimal growth can be obtained from less fortified milk because the total consumed volume of milk is increased. In this case, higher dilution factors (e.g. 1:9 and after even 1:19 in the final volumes of the fortifier to the human milk) would be used. The role of the protein fortification is gradually decreasing with gestational age of the neonate in the case of optimal growth and weight gain. A possible treatment regime is shown in the following table, listing the average daily volumes of human milk and the inventive fortifier for a neonate with a birth weight of 0.8 kg, assuming that the protein concentration of human milk (HM) is 10 mg/ml, whereas the protein concentration of the used fortifier is 100 mg/ml.

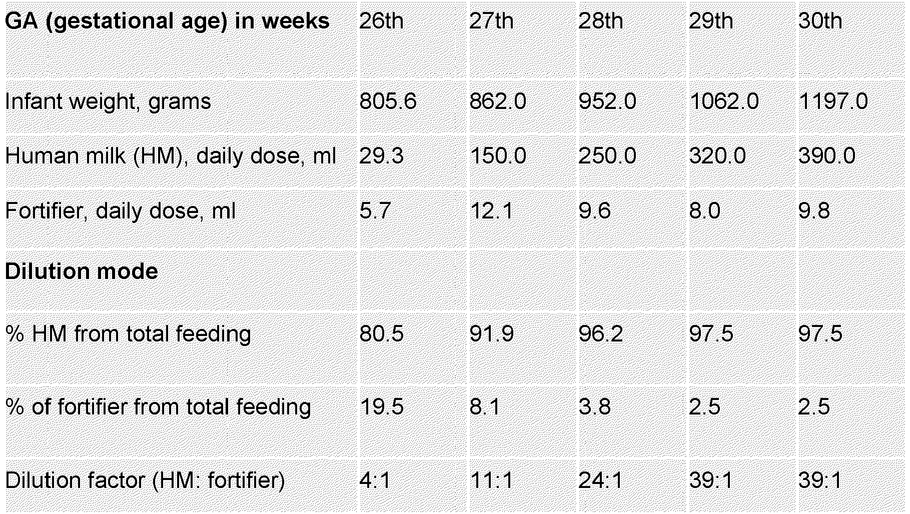

IThe method may comprise freezing the human milk fortifier. The method may comprise freezing the human milk fortifier and thawing the human milk fortifier. The method may comprise feeding the human milk fortifier to the neonate within 48 hours if the protein-enriched retentate is stored at about 4 C. The method may comprise freezing the human milk fortifier, thawing the human milk fortifier, and feeding the human milk fortifier to the neonate after dilution with human milk. The human milk fortifier may be not frozen and thawed. The human milk fortifier may be prepared between two and five times during the total time of infant hospitalization after birth.

Reference to "about" a value or parameter herein includes (and describes) embodiments that are directed to that value or parameter per se. For example, description referring to "about X" includes description of "X". In some embodiments, the term "about" when used in association with a measurement, or used to modify a value, a unit, a constant, or a range of values, refers to variations of +/- 10%, 5%, 2% or 1%.

Reference to "between" two values or parameters herein includes (and describes) embodiments that include those two values or parameters per se. For example, description referring to "between x and y" includes description of "x" and "y" per se.

### EXAMPLES

### Example 1: Exemplary Preparation of Human Milk Origin Fortifier Composition

The present example details an exemplary method for the preparation of a human milk fortifier composition.

With reference to FIG. 1A, an initial human milk sample is passed through a first filtration unit containing a microfilter (1.0 µm ordered from Whatman, GE Healthcare, catalogue number 12.1821.06), with positive air pressure, to give a fat-enriched retentate and a filtrate containing protein. The filtrate obtained from the first filter is passed through a second filtration unit containing an ultrafilter (10 kDa), with positive air pressure to give a protein-enriched retentate. The filtrate obtained from the ultrafiltration contains water and other small molecules.

The protein-enriched retentate is isolated as a liquid human milk origin fortifier.

### Example 2: Preparation of Human Milk Origin Fortifier Composition

Various human milk fortifier compositions were prepared initial milk samples according to the description and using the device as described above in Example 1, using a glass fiber microfilter (1.0 µm) and a PES ultrafilter (10kDa). The non-pasteurized human breast milk (50ml total volume) was filtered using a 1.0 micron glass fiber filter and a maximum stirring speed of 200 rpm. Before the enrichment, the initial material was taken for SDS gel analysis (sample 1 labelled as U1 in Fig.1B). The pressure was maintained between 0.2-0.5 bar for the initial 5 minutes of filtration and subsequently increased to 3 bar. The sample was concentrated by a factor of 10 in the first filtration step to result in 5.0 mL of retentate (sample 2 labelled as U2 in Fig.1B). 1.0 mL of the thick liquid fat-enriched fraction was collected for SDS gel. The separation of fat was good since the liquid was very transparent compared with the non-transparent initial milk. Ca 45 mL of the filtrate (sample3 labelled as U3 in Fig.1B).) was collected for SDS gel as well. The protein loss during the fat removal step was not high (comparing the intensity of the staining of the protein bands of U1 and U3). Based on the SDS gel results, protein lost during the fat removal step could be around 50% of the initial total protein amount). Fat-reduced filtrate (sample 3 or U3) was then filtrated through a 10 kDa ultrafilter made from polyethersulfone (PES) at a maximum pressure of 4 bar and a maximum stirring speed of 200 rpm. The solution was concentrated by a factor of ca 4 to result in 8mL of a protein-containing retentate (sample 4, U4). This filtrate was also collected (sample 5, U5). Based on SDS gel result (comparing U4 with U1), protein concentration was efficient.

The efficiency of the protein enrichment was analyzed by gel electrophoresis (SDS-PAGE) and by BCA (bicinchoninic acid) assay to measure exact protein content.

*BCA protein* assay *sample preparation:* 50 µL aliquots of each sample were taken and combined with a solution of 2% sodium dodecyl-sulfate (SDS) in water. Each sample mixture was centrifuged for 60 minutes at 1000 rpm and at 4°C. After centrifugation, a further 10 µL aliquot was isolated from the centrifuged sample, diluted (1/10 dilution) and subjected to BCA analysis. Samples were pre-treated before application of BCA to remove the lipids (several protocols were applied and most efficient was selected).

*Sodium Dodecyl-Sulfate Polyacrylamide Gel Electrophoresis sample preparation:* 5 µL aliquots of each sample were taken and combined with 5 µL water and 10 µL Laemmli buffer. Each sample mixture was boiled for 5 minutes at 95°C. The boiled sample mixtures were then loaded onto a 12.5% SDS-PAGE gel. The resulting gels were stained/blotted with a Coomassie staining solution.

Most of the collected samples were subjected to SDS-PAGE. The results obtained from SDS gel and BCA data were also confirmed by the Kjeldahl method, which was found to be in agreement with the data.

FIG. 1B shows the results of protein analysis using SDS-PAGE and protein staining at various stages of the two-step filtration process, as depicted in FIG. 1A. In FIG. 1B, the various lanes correspond to: #1, initial milk sample; #2, fat-enriched fraction (retentate from microfiltration at 1.0 µm); #3, fat-reduced filtrate from microfiltration; #4, protein-enriched retentate (HMOF) (after ultrafiltration at 10 kDa); and #5, filtrate from ultrafiltration. As shown in FIG. 1B, lane #4, substantial protein content is preserved and concentrated in the retentate obtained from ultrafiltration.

Table 1 shows the results of a bicinchoninic acid (BCA) colorimetric protein assay for determining the protein content of the initial milk sample to the resulting human milk origin fortifier composition.

**Table 1. BCA Measurement of Protein Concentration**

| **Fractions** | **Description** | **Volume (mL)** | **Protein Concentration (mg/mL)** |
|---|---|---|---|
| **Initial milk sample** | Human milk (not filtered) | 50 | 8.7 |
| **Human Milk Origin Fortifier (HMOF)** | Protein-enriched sample (obtained from two step filtration, 1.0 µM and 10 kDa) | 12.5 | 16.9 |

In addition to analysis of the total protein content, the initial milk samples and resulting human milk origin fortifier compositions were analyzed by mass spectrometry for proteomic evaluation. Based on the results below, the data for which were reproducible for several set of independent enrichment procedures, it was observed that enrichment protocol provides generally no biased protein distribution before and after protein enrichment. Thus, the resulting protein distributions of the HMOF compositions prepared by the devices and methods described herein would be very similar to the distribution of proteins in the non-treated human milk.

**Table 2. Proteomic Data Obtained for Initial Milk and Enriched Protein Fraction (Protein HMOF).**

| **n** | **Protein** | **Relative concentration of 25 most abundant proteins (in %)** | |
|---|---|---|---|
| | | **Initial milk sample** | **Human Milk Origin Fortifier (HMOF)** |
| 1 | Beta-Casein | 25.0 | 23.0 |
| 2 | Kappa-Casein | 14.5 | 11.6 |
| 3 | Lactotransferrin | 12.0 | 9.4 |
| 4 | Alpha-lactalbumin | 7.0 | 8.0 |
| 5 | Immunoglobulin (Heavy Constant Alpha-1) | 5.2 | 7.7 |
| 6 | Isoform 3 of Alpha-S1-casein | 7.4 | 4.9 |
| 7 | Immunoglobulin lambda constant 2 | 3.0 | 4.7 |
| 8 | Immunoglobulin kappa light chain | 2.1 | 4.3 |
| 9 | Isoform 2 of Alpha-S1-casein | 4.4 | 3.6 |
| 10 | Bile salt-activated lipase O | 4.0 | 2.9 |
| 11 | Polymeric immunoglobulin receptor | 1.8 | 2.7 |
| 12 | Immunoglobulin J chain | 1.0 | 1.8 |
| 13 | Albumin | 2.1 | 1.4 |
| 14 | Isoform D of Osteopontin | 0.4 | 0.9 |
| 15 | Fatty acid-binding protein | 0.4 | 0.8 |
| 16 | Zinc-alpha-2-glycoprotein | 0.3 | 0.8 |
| 17 | Xanthine dehydrogenase/oxidase | 0.2 | 0.7 |
| 18 | Immunoglobulin kappa constant | 0.8 | 0.6 |
| 19 | Immunoglobulin alpha-2 heavy chain | 0.3 | 0.6 |
| 20 | Immunoglobulin gamma-1 heavy chain | 0.3 | 0.6 |
| 21 | Macrophage mannose receptor 1 | 0.1 | 0.5 |
| 22 | Lysozyme C | 0.2 | 0.5 |
| 23 | Isoform 6 of Clusterin | 1.4 | 0.5 |
| 24 | Butyrophilin | 0.8 | 0.4 |
| 25 | Monocyte differentiation antigen CD14 | 0.1 | 0.3 |
| 26 | Other proteins with minor abundance (<300) | 4.6 | 6.7 |

### Example 2: Evaluation of Various Microfiltration Flat Sheet Filters for Fat Removal

The present example details the evaluation of various filters having different pore sizes and/or made of different filter materials on the fat removal (retention) and protein permeation (collection) concentration from an initial human breast milk sample.

The experiment has been performed using Amicon^{®} stirring cells that are commercially available from Merck KGaA, Darmstadt, Germany as well as with filters having a diameter of 76mm. The used cells have a capacity of 0.4L maximum compared to the maximum volume capacity of 2.0L of the inventive device described above. Accordingly, using the commercial stirring cells, the amount of milk required for the experiments could be greatly reduced compared to performing the experiments with the inventive device.

The diameter of the filter of the inventive device described above is 170 mm and therefore, the total area of the filter is 227cm². The total area of the filters used in the stirring cells is 45.3cm², i. e. 3.8 times less. It may be assumed that using the inventive device the total time of filtrating the same volume of initial material (human milk) at the same applied pressure would be reduced by at least 2-3 times.

The main aim of these experiments related to the optimum choice of filter material. The results related to the filter performance in order to efficiently remove the fat fraction (first filter) and in order to concentrate the protein fraction (second filter) can be extrapolated to the inventive device. The pressure to be applied should be comparable, due to having a larger interface between the filter and the liquid, the pressure might even be reduced in the context of the inventive device.

The filtering steps occurred at room temperature. Filtering times depended on the sample volume and the type of filter; they ranged from 0.5-5 h. Filters used in the inventive process should comply with the relevant standards, such as e. g. EC 1935/2004 and EC 10/2011 for plastics materials.

SDS-PAGE was used to study the protein content of samples. In each case, a sample volume of 1 ml was prepared and about 50 µl have been used for the actual SDS gel line. All samples were collected as fresh as possible and kept at -18 °C before the SDS gel run.

**Trial 1:** Non-pasteurized human breast milk (sample 1, S1) was used during the experiments in Trial 1 to Trial 4. For Trial 1 the breast milk (35ml total volume) was filtered using a 1.0 micron glass fiber filter and a maximum stirring speed of 200 rpm. The pressure was maintained between 0.2-0.5 bar for the initial 5 minutes of filtration, increased to 0.2-1 bar for 1 hour, and subsequently increased to 3 bar. The sample was concentrated by a factor of 10 in the first filtration step to result in 3.5 mL of retentate. 0.8 mL of the thick liquid fat-enriched fraction was collected (sample 2, S2). The separation of fat was good since liquid was very transparent compared with non-transparent initial milk. 25 mL of the filtrate (sample 3, S3) was collected for SDS gel as well. The protein loss during fat removal step was not high (comparing the S1 and S3 and seeing the intensity of the staining of the protein bands). Based on the SDS gel results we assume that protein lost during fat removal step could be less 50% from initial total protein amount). Fat-reduced filtrate (sample 3 or S3) was filtrated through a 10 kDa ultrafilter from polyethersulfone (PES) at a maximum pressure of 4 bar and a maximum stirring speed of 200 rpm. The solution was concentrated by a factor of 5 to result in 5 mL of a protein-containing retentate (sample 4, S4). This retentate was further concentrated by a factor of 2 to result in 2.5 mL of a protein-containing retentate (sample 5, S5). Based on SDS gel result we conclude that protein concentration was efficient (S4 and S5 comparing with S1). The protein loss during protein concentration step was not detected since we do not observe a decrease of protein (comparing the S4 and S5 and seeing the intensity of the staining of the protein bands). Based on the SDS gel results we assume that protein lost during protein concentration step could be less 10% from initial total protein amount). Thus, most of protein lost occurred during first step when we remove the fat fraction.

**Trial 2:** 35 mL of non-pasteurized human breast milk (initial material was the same as sample 1, S1) was filtered at 0.1 bar using a 0.2-0.4 µm cellulose filter and a maximum stirring speed of 200 rpm.

25 mL of a white filtrate was collected. The retentate was foamy and fat (sample 6, S6) while the filtrate was pretty transparent (sample 7, S7), both samples were collected for SDS gel.

The separation of fat was partial since the liquid was not so transparent compared with the fat-removed filtrate from Trial 1 (S3). The protein loss during the fat removal step was not high (comparing the S1 and S7 and seeing the intensity of the staining of the protein bands). Based on the SDS gel results, the protein lost during the fat removal step could be 50% or less of the initial total protein amount).

**Trial 3:** Human breast milk (35 mL) was passed through a 0.2µm polycarbonate membrane filter at a maximum pressure of 1 bar and a maximum stirring speed of 200 rpm. After 50 minutes, 20 mL of filtrate had passed through the filter. The pressure was increased to 2 bars and the filtration was stopped when the retentate was 5ml. 5 mL of retentate (sample 9, S9) and 25 mL of transparent filtrate (sample 10, S10) were collected.

The separation of fat was very efficient since the liquid (sample 10, S10) was very transparent compared with the non-transparent initial milk. 25 mL of the filtrate (sample 10, S10) was collected for SDS gel. The protein loss during the fat removal step was very high (comparing the S1 and S10 and seeing the intensity of the staining of the protein bands). Based on the SDS gel results, the protein lost during the fat removal step was more than 80% of the initial total protein amount).

Trial 4: Human breast milk (35 mL), same as sample 1 (S1) was passed through a 0.2µm polyvinylidene fluoride (PVDF) filter at a pressure of 3-4 bar and a maximum stirring speed of 200 rpm. 5 mL of a fat retentate and 30 mL of a transparent filtrate were collected (sample 11, S11, and sample 12, S12, respectively).

The separation of fat was very efficient since the liquid (sample 12, S12) was very transparent compared with the non-transparent initial milk. 30 mL of the filtrate (sample 12, S12) was collected for SDS gel. The protein loss during fat removal step was very high (comparing the S1 and S12 and seeing the intensity of the staining of the protein bands). Based on the SDS gel results, the protein lost during the fat removal step was more than 90% of the initial total protein amount).

**Trial 5:** A fresh sample of human breast non-pasteurized milk (35 mL, sample 13, S13) was collected and passed through a 0.1µm cellulose filter at 0.5 bar with stirring. Within one hour, a fat retentate (sample 14, S14) and 20 mL of filtrate (sample 15, S15) were collected. The separation of fat was very efficient since the liquid (sample 15, S15) was very transparent compared with the non-transparent initial milk. 20 mL of the filtrate (sample 15, S15) was collected for SDS gel. The protein loss during fat removal step was very high (comparing the S13 and S15 and seeing the intensity of the staining of the protein bands). Based on the SDS gel results, the protein lost during fat removal step was more than 90% of the initial total protein amount).

**Trial 6:** Human breast milk (17 mL, same as sample 13, S13) was passed through a 0.2 µM nitrocellulose filter at a pressure of less than 1 bar with stirring. A fat retentate, volume 7ml (sample 16, S16) and a fat-reduced filtrate, volume 10ml (sample 17, S17) were collected.

The separation of fat was very efficient since the liquid (sample 17, S17) was quite transparent compared with the non-transparent initial milk. 20 mL of the filtrate (sample 15, S15) was collected for SDS gel. The protein loss during fat removal step was not so high (comparing the S13 and S17) by seeing the intensity of the staining of the protein bands. Based on the SDS gel results, the protein lost during the fat removal step was more than 50% but less than 70% of the initial total protein amount).

**Trial 7:** Human pasteurized breast milk (60 mL, sample 18 or S18) was passed through a 0.2-0.4 µM cellulose filter at a pressure of less than 0.5 bar with stirring. A filtration was stopped when retentate reached 5ml of total volume. The fat-reduced filtrate (sample 19, S19) at the total volume 55ml was collected. The separation of fat was quite good since the liquid was less fatty than the initial milk. The protein loss during fat removal step was not high (comparing the S18 and S19 and seeing the intensity of the staining of the protein bands. Based on the SDS gel results, the protein lost during fat removal step could be less than 20% of the initial total protein amount).

The fat-reduced sample (S19) was then concentrated (protein-enriched) by using cellulose filter with pore size 100nanoM (ca >500kDa pore size). 25ml were passed through a 100nanoM filter at a pressure of less than 0.5 bar. The concentration finished when 5ml of retentate left. A protein-containing sample (retentate), total volume of 5ml (sample 20, S20) was collected for SDS gel.

Based on the SDS gel results (FIG.4A) the protein enrichment with the cellulose filter with a large pore size (100nanoM) was not good since the sample contains only a very small amount of proteins. The pore size was too big to concentrate the proteins and most of proteins went to the filtrate (not collected). For the efficient protein enrichment the pore size of 100kDa or less should be used.

**Trial 8:** Human breast milk (60 mL, same material as was used for Trial 7, sample 18 or S18) was passed through a 0.2 µM mixed cellulose esters (MCE) filter. The milk was passed through the filter at a pressure of 4 bar with stirring. A fat-containing retentate was kept at volume of 5ml and the fat-reduced filtrate (sample 21, S21) at the total volume 50ml was collected. The separation of fat was good since liquid was very transparent. The protein loss during fat removal step was very high (comparing the S18 and S21 and seeing the intensity of the staining of the protein bands. Based on the SDS gel results we assume that protein lost during fat removal step could be more than 70% from initial total protein amount). After the fat-reduced sample (S21) was concentrated (protein-enriched) by using polyethersulfone (PES) filter with pore size 4kDa. 50ml were passed through a the 4kDa filter at a pressure of less than 4 bar with stirring. The protein concentration finished when 5ml of retentate left. A protein-containing sample (retentate), total volume of 5ml (sample 26, S26) was collected for SDS gel. Based on the SDS gel results (FIG.4A-4B) the protein enrichment with the MCE filter for the first step was not was good since initially before protein enrichment the sample contained only a very small amount of proteins, ca 20-30% from initial total amount. For protein enrichment (S26) the 4kDa pore size works efficient since all protein which remained after first step /fat removal with MCE) was sufficiently enriched (S21 and S26).

**Trial 9:** Human breast milk (120 mL, same material as was used for Trial 7, sample 18 or S18) was divided to two parts, 60ml each. The milk was passed through a 0.2-0.4 µM cellulose filter with pressure less than 0.5 bars with stirring during first ten minutes. After we started gradually increase the pressure till 2bars in total. The liquid (filtrate) was semi-transparent compared to initial milk. When remained retentate was ca 5ml, the filtration was stopped, ca 50ml of fat-reduced filtrate was obtained. We combined the obtained fat-reduced filtrate (100ml) and from there we collected 1ml of the sample for SDS gel (sample 22 or S22). The rest of the filtrate was applied to the second filtration step (protein concentration) with polyethersulfone (PES) filter with pore size 4kDa. 100ml were passed through a the 4kDa filter at a pressure of less than 4 bar with stirring. The protein concentration finished when 5ml of retentate left. A protein-containing sample (retentate), total volume of 5ml (sample 25, S25) was collected for SDS gel.

Based on the photo of the SDS samples (FIG.4C) the fat removal with the cellulose filter for the first step was still efficient since the filtrate was partially -transparent. The increase of the pressure from 0.5 to 1bar also negatively affect the efficiency of the fat separation (more fat could pass through the filter when we increased the pressure compared with the Trial 7 and sample S19, which occurred to be more transparent, Fig.4C, even if was filtrated with the same type of the filter). At the same time the fat-reduced sample contains almost the same amount of proteins as initial milk (S18 compared with S22). We can conclude seeing the intensity of the protein Coomassie-stained bands that more than 80-85% from initial total protein amount was preserved.

For protein enrichment (S25) the 4kDa pore size works efficient since all amount of the proteins was sufficiently enriched (S25 compared with the initial human milk S18).

**Trial 10:** Human breast milk (25 mL, same material as was used for Trial 7, sample 18 or S18) was passed through a 0.2 µM polyethersulfone (PES) filters. The milk was passed through the filter at a pressure of 1 bar with stirring.

The fat removal step with PES filter was very efficient since the liquid (filtrate) was very transparent compared to initial milk. When remained retentate was ca 7.5ml, the filtration was stopped, ca 16ml of fat-reduced filtrate was obtained, the rest of the sample was lost during the enrichment. From fat-reduced filtrate we collected 1ml of the sample for SDS gel (sample 23 or S23). Based on the results (FIG.4C) the fat removal with the PES filter for the first step was very efficient since the liquid was very transparent. At the same time the fat-reduced sample contains a reduced total protein content as initial milk (S18 compared with S23), ca 40-50% of protein content was lost from initial total amount of protein (FIG4A).

**Trial 11:** Human breast milk (25 mL, same material as was used for Trial 7, sample 18 or S18) was passed through a 0.2 µM cellulose acetate (CA) filters. The milk was passed through the filter at a pressure of 1 bar with stirring.

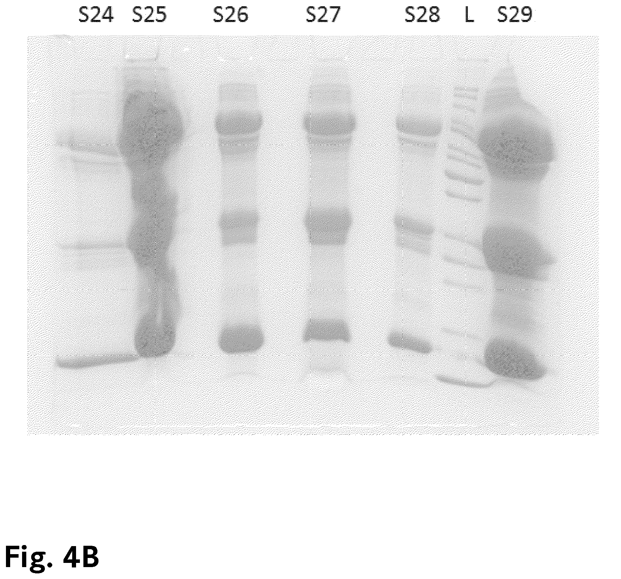

The fat removal step with CA filter was very efficient since the liquid (filtrate) was very transparent compared to initial milk. When remained retentate was ca 7.5ml, the filtration was stopped, ca 16ml of fat-reduced filtrate was obtained, the rest of the sample was lost during the enrichment. From fat-reduced filtrate we collected 1ml of the sample for SDS gel (sample 24 or S24, FIG4B). Based on the results (FIG.4C) the fat removal with the CA filter for the first step was very efficient since the liquid was very transparent. At the same time the fat-reduced sample contains a reduced total protein content as initial milk (S18 compared with S24, FIG4A-4B), more than 80% of total protein content was lost (FIG4A-4B).

**Trial 12:** Human pasteurized breast milk (120 mL, sample 27 or S27) was divided to two parts, 60ml each. Each part was passed through a 0.2-0.4 µM cellulose filter with pressure less than 0.5 bars with stirring during. The liquid (filtrate) was semi-transparent compared to initial milk. When remained retentate was ca 5ml, the filtration was stopped, ca 50ml of fat-reduced filtrate was obtained. We combined the obtained fat-reduced filtrate (100ml) and from there we collected 1ml of the sample for SDS gel (sample 28 or S28). The rest of the filtrate was applied to the second filtration step (protein concentration) with polyethersulfone (PES) filter with pore size 4kDa. 100ml were passed through a the 4kDa filter at a pressure of less than 4 bar with stirring. The protein concentration finished when 5ml of retentate left. A protein-containing sample (retentate), total volume of 5ml (sample 29, S29) was collected for SDS gel.

Based on the SDS gel results (FIG.4B) and data from Trial 2 and 7 the fat removal with the cellulose filter for the first step was pretty efficient since the liquid (fat-reduced filtrate) was almost transparent. At the same time the fat-reduced sample contains almost the same amount of proteins as initial milk (S27 compared with S28). The decrease of pressure to less than 0.5bar during first filtration step (fat removal) positively affect the fat removal process (FIG.4C). At the same time proteins are efficiently separated from the fat since more than 80% from initial total amount was preserved (FIG.4B).

Regarding protein concentration step the protein fraction was efficiently enriched (S27 and S29, FIG4B) by using 4kDa PES filter. These data in agreement with the data from Trials 8 and 9.

Samples from each trial were analyzed visually and by SDS-PAGE to assess the protein loss during first filtration step and the efficiency of the protein enrichment during second filtration step for each filter material.

Table 3 summarizes the parameters for the microfiltration filter media used for the first step, the filter's pore size, as well as the positive pressure applied during filtration. Aliquots of the initial milk, the fat enriched retentate, fat-reduced filtrate and the protein-containing retentate were taken from each trial and most of them were analyzed by SDS gel. Each aliquot is identified by a Sample ID as shown in Table 3.

**Table 3. Filters and process parameters**

| **Tria I No.** | **Microfilter Filter Material, Pore Size** | **Parameter s** | **Initia I Milk** | **Fat Enriched Retentat e** | **Protein-containin g Filtrate** | **Protein-enriche d retentat e** |
|---|---|---|---|---|---|---|
| 1 | Glass Fiber, 1 µm | 0.2-1 bar | S1 | S2 | S3 | |
| 2 | Cellulose, 0.2-0.4 µm | 0.1 bar | S1 | S6 | S7 | |
| 3 | Polycarbonate (PC), 0.2 µm | 1-2 bar | S1 | S9 | S10 | |
| 4 | Polyvinylidene fluoride (PVDF), 0.2 µm | 3-4 bar | S1 | S11 | S12 | |
| 5 | Cellulose, 0.1 µm | 0.5 bar | S13 | S14 | S15 | |
| 6 | Nitrocellulose (NC), 0.2 µm | 1 bar | S13 | S16 | S17 | |
| 7 | Cellulose, 0.2-0.4 µm | 0.5bar | S18 | | S19 | S20* |
| 8 | Mixed cellulose esters (MCE), 0.2µm | 4 bar | S18 | | S21 | S26 |
| 9 | Cellulose, 0.2-0.4 µm | 0.5-2bar | S18 | | S22 | S25 |
| 10 | Polyethersulfone (PES), 0.2µm | 1bar | S18 | | S23 | |
| 11 | Cellulose acetate (CA), 0.2µm | 1bar | S18 | | S24 | |
| 12 | Cellulose, 0.2-0.4 µm | <0.5bar | S27 | | S28 | S29 |

| | | | | | | |
|---|---|---|---|---|---|---|
| *Protein enrichment (S20) in Trial 7 had been failed there since the ultra-filter pore size was too high and most proteins went to the filtrate, see for more details Trial 7 detailed description | | | | | | |

FIGS. 3A-3C and 4A-4B depict images of the results from gel electrophoresis. Table 4 summarizes the correspondence between the various samples (as identified by sample ID) taken from various trials and the images of the gels by figure number and lane number in the gel. FIG. 3D and 4C depicts images of aliquots of the various samples taken from the trials and labelled by their respective sample ID.

**Table 4.**

| **Trial No.** | **Microfiltration Filter Material, pore size (1^{ST} filtration step)** | **FIG. No.** | **Lane** | **Sample ID** | **Description** |
|---|---|---|---|---|---|
| | | 3A | 1 | L | Protein Std. Thermo 26614 |
| 1 | Glass Fiber, 1.0µm | 3A | 2 | S1 | Initial milk |
| | | 3A | 3 | | |
| 1 | Glass Fiber, 1.0µm | 3A | 4 | S2 | Fat-enriched retentate |
| | | 3A | 5 | | |
| 1 | Glass Fiber, 1.0µm | 3A | 6 | S3 | Fat-reduced protein-containing filtrate |
| | | 3A | 7 | | |
| 1 | Glass Fiber, 1.0µm | 3A | 8 | S4 | Protein-containing retentate (after 10kDa ultrafiltration) |
| | | 3A | 9 | | |
| 1 | Glass Fiber, 1.0µm | 3A | 10 | S5 | Protein-containing retentate (after 10kDa ultrafiltration) |
| 2 | Cellulose, 0.2-0.4µm | 3B | 1 | S6 | Fat-enriched retentate |
| | | 3B | 2 | | |
| 2 | Cellulose, 0.2-0.4µm | 3B | 3 | S7 | Fat-reduced protein-containing filtrate |
| | | 3B | 4 | | |
| 3 | Polycarbonate (PC), 0.2µm | 3B | 5 | S9 | Fat-enriched retentate |
| | | 3B | 6 | | |
| 3 | Polycarbonate (PC), 0.2µm | 3B | 7 | S10 | Fat-reduced protein-containing filtrate |
| | | 3B | 8 | | |
| 4 | polyvinylidene fluoride (PVDF), 0.2µm | 3B | 9 | S11 | Fat-enriched retentate |
| | | 3B | 10 | L | Protein Std. Thermo 26614 |
| 4 | polyvinylidene fluoride (PVDF), 0.2µm | 3C | 1 | S12 | Fat-reduced filtrate |
| | | 3C | 2 | | |
| 5 | Cellulose, 0.1µm | 3C | 3 | S13 | Initial milk |
| | | 3C | 4 | | |
| 5 | Cellulose, 0.1µm | 3C | 5 | S14 | Fat-enriched retentate |
| | | 3C | 6 | | |
| 5 | Cellulose, 0.1µm | 3C | 7 | S15 | Fat-reduced filtrate |
| | | 3C | 8 | | |
| 6 | Nitrocellulose (NC), 0.2µm | 3C | 9 | S17 | Protein-containing filtrate |
| | | 3C | 10 | L | Protein Std. Thermo 26614 |
| 7 | Cellulose, 0.2-0.4µm | 4A | 1 | S18 | Initial milk |
| | | 4A | 2 | | |
| 7 | Cellulose, 0.2-0.4µm | 4A | 3 | S19 | Fat-reduced filtrate |
| | | 4A | 4 | | |
| 7 | Cellulose, 0.2-0.4µm | 4A | 5 | S20 | Protein-containing retentate (after 100nanoM ultrafiltration) |
| | | 4A | 6 | | |
| 8 | Mixed cellulose esters (MCE), 0.2µm | 4A | 7 | S21 | Fat-reduced protein-containing filtrate |
| | | 4A | 8 | L | Protein Std. Thermo 26614 |
| 9 | Cellulose, 0.2-0.4µm | 4A | 9 | S22 | Fat-reduced protein-containing filtrate |
| 10 | Polyethersulfone (PES), 0.2µm | 4A | 10 | S23 | Fat-reduced protein-containing filtrate |
| 11 | Cellulose acetate (CA), 0.2µm | 4B | 1 | S24 | Fat-reduced protein-containing filtrate |
| 9 | Cellulose, 0.2-0.4µm | 4B | 2 | S25 | Protein-containing retentate (after 4kDa ultrafiltration) |
| | | 4B | 3 | | |
| 8 | Mixed cellulose esters (MCE), 0.2Um | 4B | 4 | S26 | Protein-containing retentate (after 4kDa ultrafiltration) |
| | | 4B | 5 | | |
| 12 | Cellulose, 0.2-0.4µm | 4B | 6 | S27 | Initial milk |
| | | 4B | 7 | | |
| 12 | Cellulose, 0.2-0.4µm | 4B | 8 | S28 | Fat-reduced protein-containing filtrate |
| | | 4B | 9 | L | Protein Std. Thermo 26614 |
| 12 | Cellulose, 0.2-0.4µm | 4B | 10 | S29 | Protein-containing retentate (after 4kDa ultrafiltration) |

The trials show that the same filter pore size can provide different results depending on the filter material, post treatment of the filter like etching, additional modifications like absence or presence of a binder (in case of e. g. glass fiber filters), etc. Nevertheless, the trials demonstrate some filters successfully remove fat (first filter) with low absorption of proteins and concentrate the protein fraction (second filter). In particular, the glass-fiber filter with a pore size of 1.0 µm efficiently removed fat globules from human milk at relatively low pressure values of 2 bars or less. This can bee seen from the resulting liquid which looked transparent and almost fat-free compared with the initial human milk. This filtering step reduced the total amount of proteins by about 50%. In order to avoid contamination of the fat-reduced protein-containing filtrate by glass fiber material suitable further filters may be employed downstream the glass fiber filter.

Such additional filtering may be avoided if other filter materials such as cellulose are used. The examined cellulose filters with a pore size of 0.2-0.4µm have shown to be less efficient with respect to fat removal from human milk, but at the same time the protein loss was reduced. In this respect, it is important to note that the three-dimensional structure of cellulose filter material is more complex than that of most synthetic filters. Therefore, milk fat globules being different in size can be more efficiently caught. Indeed, the trials have shown that the performance of the cellulose filters for fat removal was better than that of filters from other materials such as NC, CA, MCE or PES.

This is confirmed by the finding that a decreased pressure provides better separation as this allows the process of fat removal to become slower and allow the fat globules to equilibrate the filter material and be caught inside the filters more efficiently.

## Claims

1. A device for protein enrichment of human breast milk, comprising:
a delipidation unit, comprising
a first reservoir configured to receive breast milk, wherein the first reservoir is connected to a first filter;
a first filter, wherein the first filter is in fluid connection with the first reservoir and is a cellulose filter having a pore size of 0.2-0.4 µm, wherein the first filter is configured to receive breast milk from the first reservoir and configured to provide a fat-enriched retentate and a fat-reduced filtrate from the breast milk;
a pressure valve, wherein the pressure valve is in fluid connection with the first reservoir and the first filter, and wherein the pressure valve is configured to provide positive air pressure to the delipidation unit; and
an output wherein the output is configured to deliver the fat-reduced filtrate provided by the first filter;
a protein-enrichment unit, wherein the protein-enrichment unit comprises:
a second reservoir configured to receive the fat-reduced filtrate from the output of the delipidation unit; and
a second filter, wherein the second filter has a nominal molecular weight limit of less than or equal to 100 kDa, in particular of less than or equal to 10 kDa, and wherein the second filter is configured to provide a protein-enriched retentate and a protein-reduced filtrate.

2. The device of claim 1, wherein the device is configured to maintain a temperature of between about 4 and about 10 degrees Celsius.

3. The device of any of the preceding claims, wherein the pressure valve is configured to supply between about 0.5 bar and about 4 bar of absolute pressure to the first reservoir and first filter.

4. The device of any of the preceding claims, wherein the pressure valve is configured to supply between about 0.1 bar and about 4 bar of absolute pressure to the second reservoir and second filter.

5. The device of any of the preceding claims, wherein the device comprises a second pressure valve, wherein the second pressure valve is in fluid connection with the second reservoir and the second filter, wherein the second pressure valve is configured to provide positive air pressure to the protein enrichment unit.

6. The device of claim 5, wherein the second pressure valve is configured to supply between about 0.5 and about 4 bar of absolute pressure to the second reservoir and second filter.

7. The device of any of the preceding claims, wherein the pressure valve and/or the second pressure value is configured to provide air pressure.

8. The device of any of the preceding claims, wherein the protein enrichment unit further comprises a second output and a third reservoir, wherein the second output is configured to deliver the protein-reduced filtrate to the third reservoir.

9. The device of any of the preceding claims, wherein the delipidation unit is configured to agitate and/or stir breast milk in the first reservoir and/or wherein the protein enrichment unit is configured to agitate and/or stir breast milk in the second reservoir.

10. A method for preparing human milk fortifier, using a device according to any of the preceding claims, the method comprising:
passing human breast milk through a first filter and collecting a fat-reduced filtrate; and
passing the fat-reduced filtrate through a second filter and collecting a protein-enriched retentate, wherein the method does not employ chemical means, thermal means or centrifugation to remove fat from human breast milk prior to concentration of the protein.

11. The method of claim 10, wherein the temperature of the human breast milk is between about 4 and about 10 °C.

12. The method of claim 10 or 11, wherein the volume of the human milk fortifier is between about 10 and about 20 times less than the volume of the human breast milk.

## Patentansprüche

1. Eine Vorrichtung zur Proteinanreicherung von menschlicher Muttermilch, umfassend: eine Delipidierungseinheit, umfassend einen ersten Behälter, der so konfiguriert ist, dass er Muttermilch aufnimmt, wobei der erste Behälter mit einem ersten Filter verbunden ist; einen ersten Filter, wobei der erste Filter in Fluidverbindung mit dem ersten Behälter steht und ein Zellulosefilter mit einer Porengröße von 0,2-0,4 µm ist, wobei der erste Filter so konfiguriert ist, dass er Muttermilch aus dem ersten Behälter aufnimmt und ein fettreiches Retentat sowie ein fettarmes Filtrat aus der Muttermilch liefert; ein Druckventil, wobei das Druckventil in Fluidverbindung mit dem ersten Behälter und dem ersten Filter steht und so konfiguriert ist, dass es positiven Luftdruck auf die Delipidierungseinheit ausübt; und einen Ausgang, wobei der Ausgang so konfiguriert ist, dass er das vom ersten Filter bereitgestellte fettarme Filtrat liefert; eine Proteinanreicherungseinheit, wobei die Proteinanreicherungseinheit umfasst: einen zweiten Behälter, der so konfiguriert ist, dass er das fettarme Filtrat aus dem Ausgang der Delipidierungseinheit aufnimmt; und einen zweiten Filter, wobei der zweite Filter eine nominale Molekulargewichtsgrenze von maximal 100 kDa, insbesondere von maximal 10 kDa, aufweist und so konfiguriert ist, dass er ein proteinreiches Retentat und ein proteinreduziertes Filtrat liefert.

2. Die Vorrichtung nach Anspruch 1, wobei die Vorrichtung so konfiguriert ist, dass sie eine Temperatur zwischen etwa 4 und etwa 10 Grad Celsius aufrechterhält.

3. Die Vorrichtung nach einem der vorhergehenden Ansprüche, wobei das Druckventil so konfiguriert ist, dass es einen absoluten Druck zwischen etwa 0,5 bar und etwa 4 bar auf den ersten Behälter und den ersten Filter ausübt.

4. Die Vorrichtung nach einem der vorhergehenden Ansprüche, wobei das Druckventil so konfiguriert ist, dass es einen absoluten Druck zwischen etwa 0,1 bar und etwa 4 bar auf den zweiten Behälter und den zweiten Filter ausübt.

5. Die Vorrichtung nach einem der vorhergehenden Ansprüche, wobei die Vorrichtung ein zweites Druckventil umfasst, wobei das zweite Druckventil in Fluidverbindung mit dem zweiten Behälter und dem zweiten Filter steht und so konfiguriert ist, dass es positiven Luftdruck auf die Proteinanreicherungseinheit ausübt.

6. Die Vorrichtung nach Anspruch 5, wobei das zweite Druckventil so konfiguriert ist, dass es einen absoluten Druck zwischen etwa 0,5 und etwa 4 bar auf den zweiten Behälter und den zweiten Filter ausübt.

7. Die Vorrichtung nach einem der vorhergehenden Ansprüche, wobei das Druckventil und/oder das zweite Druckventil so konfiguriert ist, dass es Luftdruck bereitstellt.

8. Die Vorrichtung nach einem der vorhergehenden Ansprüche, wobei die Proteinanreicherungseinheit ferner einen zweiten Ausgang und einen dritten Behälter umfasst, wobei der zweite Ausgang so konfiguriert ist, dass er das proteinreduzierte Filtrat an den dritten Behälter liefert.

9. Die Vorrichtung nach einem der vorhergehenden Ansprüche, wobei die Delipidierungseinheit so konfiguriert ist, dass sie die Muttermilch im ersten Behälter rührt und/oder bewegt, und/oder wobei die Proteinanreicherungseinheit so konfiguriert ist, dass sie die Muttermilch im zweiten Behälter rührt und/oder bewegt.

10. Ein Verfahren zur Herstellung eines Muttermilchverstärkers, unter Verwendung einer Vorrichtung nach einem der vorhergehenden Ansprüche, wobei das Verfahren umfasst: das Durchleiten menschlicher Muttermilch durch einen ersten Filter und das Sammeln eines fettarmen Filtrats; und das Durchleiten des fettarmen Filtrats durch einen zweiten Filter und das Sammeln eines proteinreichen Retentats, wobei das Verfahren keine chemischen, thermischen oder zentrifugalen Mittel zur Entfernung von Fett aus menschlicher Muttermilch vor der Proteinkonzentration einsetzt.

11. Das Verfahren nach Anspruch 10, wobei die Temperatur der menschlichen Muttermilch zwischen etwa 4 und etwa 10 °C liegt.

12. Das Verfahren nach Anspruch 10 oder 11, wobei das Volumen des Muttermilchverstärkers zwischen etwa 10- und etwa 20-mal kleiner als das Volumen der menschlichen Muttermilch ist.

## Revendications

1. Un dispositif d'enrichissement en protéines du lait maternel humain, comprenant: une unité de délipidation, comprenant un premier réservoir configuré pour recevoir du lait maternel, premier réservoir étant connecté à un premier filtre ; un premier filtre, le premier filtre étant en connexion fluide avec le premier réservoir et étant un filtre en cellulose ayant une taille de pores de 0,2-0,4 µm, le premier filtre étant configuré pour recevoir le lait maternel du premier réservoir et fournir un rétentat enrichi en matières grasses et un filtrat réduit en matières grasses à partir du lait maternel ; une valve de pression, la dite valve de pression étant en connexion fluide avec le premier réservoir et le premier filtre, et la dite valve de pression étant configurée pour fournir une pression d'air positive à l'unité de délipidation ; et une sortie, lad ite sortie étant configurée pour délivrer le filtrat réduit en matières grasses fourni par le premier filtre ; une unité d'enrichissement en protéines, la dite unité d'enrichissement en protéines comprenant : un second réservoir configuré pour recevoir le filtrat réduit en matières grasses depuis la sortie de l'unité de délipidation ; et un second filtre, le dit second filtre ayant une limite nominale de poids moléculaire inférieure ou égale à 100 kDa, en particulier inférieure ou égale à 10 kDa, et le dit second filtre étant configuré pour fournir un rétentat enrichi en protéines et un filtrat réduit en protéines.

2. Le dispositif selon la revendication 1, dans lequel le dispositif est configuré pour maintenir une température comprise entre environ 4 et environ 10 degrés Celsius.

3. Le dispositif selon l'une quelconque des revendications précédentes, dans lequel la valve de pression est configurée pour fournir une pression absolue comprise entre environ 0,5 bar et environ 4 bars au premier réservoir et au premier filtre.

4. Le dispositif selon l'une quelconque des revendications précédentes, dans lequel la valve de pression est configurée pour fournir une pression absolue comprise entre environ 0,1 bar et environ 4 bars au second réservoir et au second filtre.

5. Le dispositif selon l'une quelconque des revendications précédentes, dans lequel le dispositif comprend une seconde valve de pression, la dite seconde valve de pression étant en connexion fluide avec le second réservoir et le second filtre, ladite seconde valve de pression étant configurée pour fournir une pression d'air positive à l'unité d'enrichissement en protéines.

6. Le dispositif selon la revendication 5, dans lequel la seconde valve de pression est configurée pour fournir une pression absolue comprise entre environ 0,5 et environ 4 bars au second réservoir et au second filtre.

7. Le dispositif selon l'une quelconque des revendications précédentes, dans lequel la valve de pression et/ou la seconde valve de pression est configurée pour fournir une pression d'air.

8. Le dispositif selon l'une quelconque des revendications précédentes, dans lequel l'unité d'enrichissement en protéines comprend en outre une seconde sortie et un troisième réservoir, la dite seconde sortie étant configurée pour délivrer le filtrat réduit en protéines au troisième réservoir.

9. Le dispositif selon l'une quelconque des revendications précédentes, dans lequel l'unité de délipidation est configurée pour agiter et/ou remuer le lait maternel dans le premier réservoir et/ou dans lequel l'unité d'enrichissement en protéines est configurée pour agiter et/ou remuer le lait maternel dans le second réservoir.

10. Un procédé de préparation d'un fortifiant pour lait humain, en utilisant un dispositif selon l'une quelconque des revendications précédentes, le procédé comprenant : le passage du lait maternel humain à travers un premier filtre et la collecte d'un filtrat réduit en matières grasses ; et le passage du filtrat réduit en matières grasses à travers un second filtre et la collecte d'un rétentat enrichi en protéines, le dit procédé n'employant pas de moyens chimiques, thermiques ou de centrifugation pour retirer les matières grasses du lait maternel humain avant la concentration des protéines.

11. Le procédé selon la revendication 10, dans lequel la température du lait maternel humain est comprise entre environ 4 et environ 10 °C.

12. Le procédé selon la revendication 10 ou 11, dans lequel le volume du fortifiant pour lait humain est entre environ 10 et environ 20 fois inférieur au volume du lait maternel humain.
